# EUROPÄISCHE PATENTSCHRIFT

(11) **EP 3 319 650 B1**
(45) Veröffentlichungstag und Bekanntmachung des Hinweises auf die Patenterteilung: **06.03.2019**
(21) Anmeldenummer: 16759734.3
(22) Anmeldetag: 30.08.2016
(51) Int. Cl.: A61L 15/20, A61L 15/36

(54) **NEUARTIGER HYGIENEARTIKEL**
NOVEL HYGIENE ARTICLE
NOUVEL ARTICLE D'HYGIÈNE

(30) Priorität: 10.11.2015 DE 102015014424; 18.11.2015 DE 102015119941
(43) Veröffentlichungstag der Anmeldung: 16.05.2018
(73) Patentinhaber: Delphi Beteiligungsgesellschaft MbH, 82008 Unterhaching (DE)
(72) Erfinder: STRÖER, Tina, 50968 Köln (DE)
(74) Vertreter: Von Rohr Patentanwälte Partnerschaft mbB
(86) Internationale Anmeldenummer: PCT/EP2016/070355
(87) Internationale Veröffentlichungsnummer: WO 2017/080696

(56) Entgegenhaltungen:
- EP-A1- 1 118 340
- EP-A2- 1 880 727
- WO-A1-03/038068
- WO-A1-2008/060200
- WO-A1-2010/130541
- WO-A1-2014/184644
- DE-A1- 2 309 575
- DATABASE WPI Week 200352 Thomson Scientific, London, GB; AN 2003-551575 XP002763889, & KR 2003 0021298 A (CHOI H K) 15. März 2003 (2003-03-15)
- JAVIER HAYA ET AL: "Importance of Lactic Acid in Maintaining Vaginal Health: A Review of Vaginitis and Vaginosis Etiopathogenic Bases and a Proposal for a New Treatment", OPEN JOURNAL OF OBSTETRICS AND GYNECOLOGY, Bd. 04, Nr. 13, 1. Januar 2014 (2014-01-01), Seiten 787-799, XP055317037, US ISSN: 2160-8792, DOI: 10.4236/ojog.2014.413109

## Beschreibung

Die vorliegende Erfindung betrifft das technische Gebiet der Hygiene- und Sanitätsprodukte.

Insbesondere betrifft die vorliegende Erfindung einen Hygieneartikel, welcher für die Verwendung bei der prophylaktischen bzw. therapeutischen Behandlung von Erkrankungen des Urogenitaltrakts bzw. Windelregion geeignet ist.

Darüber hinaus betrifft die vorliegende Erfindung einen Hygieneartikel zur Verwendung bei der prophylaktischen bzw. therapeutischen Behandlung von Erkrankungen des Urogenitaltrakts bzw. der Windelregion.

Weiterhin betrifft die vorliegende Erfindung auch eine Kombination von Milchsäure produzierenden Bakterien und Milchsäure zur Verwendung bei der prophylaktischen bzw. therapeutischen Behandlung von Erkrankungen des Urogenitaltrakts und/oder der Windelregion.

Schließlich betrifft die vorliegende Erfindung auch eine Kombination ("Wirkstoffkombination"), insbesondere zur Ausrüstung eines Hygieneartikels (Sanitärartikels) mit keimhemmenden, insbesondere bakterien- und/oder pilzhemmenden Eigenschaften sowie eine Kombination ("Wirkstoffkombination") zur Verwendung bei der prophylaktischen und/oder therapeutischen Behandlung von Erkrankungen des Urogenitaltrakts und/oder der Windelregion, insbesondere Vaginalinfektionen, Ureterinfektionen, Vulvitis, Windeldermatitis und/oder Ekzemen.

Unter dem Urogenitaltrakt, synonym auch als Harn- und Geschlechtsapparat bezeichnet, werden üblicherweise die Organe, welche zur Harnproduktion beitragen, sowie die primären Geschlechtsorgane verstanden.

Im gesunden Zustand ist der Urogenitaltrakt, insbesondere der äußere Bereich, d. h. die äußeren Geschlechtsorgane sowie die sogenannte Windelregion, von zahlreichen Mikroorganismen bzw. einem komplexen Mikrobiom besiedelt. Dieses spezielle mikrobiologische Ökosystem umfasst mehr als fünfzig verschiedene Bakterienspezies. Bei Frauen im gebärfähigen Alter handelt es sich dabei insbesondere um Bakterien der Gattung *Lactobacillus,* insbesondere *L. iners, L. delbruekii, L. crispatus, L. vaginalis, L. acidophilus, L. jensenii, L. buchneri* und *L. gasseri.* Darüber hinaus enthält das natürliche Mikrobiom des weiblichen Urogenitaltrakts, synonym auch als Vaginalflora bezeichnet, sowie der umliegenden bzw. angrenzenden Windelregion Mikroorganismen der Gattung *Bifidobacterium.*

Bei den den weiblichen Urogenitaltrakt und die Windelregion besiedelnden Bakterien handelt es sich vorwiegend um Milchsäuregärung betreibende Spezies, insbesondere Milchsäurebakterien, d. h. Mikroorganismen, welche im Rahmen ihres Stoffwechsels ausgehend von Glucose und anderen Monosacchariden Milchsäure produzieren. Auf Basis der Bildung eines Biofilms durch die zahlreichen Milchsäurebakterien wird eine Kolonisierung durch andere, insbesondere pathogene Mikroorganismen verhindert. Darüber hinaus wird durch die Produktion von Milchsäure der pH-Wert im Urogenitaltrakt bzw. in der Windelregion im sauren Bereich gehalten, so dass auch auf dieser Basis einer Besiedelung durch pathogene Mikroorganismen entgegengewirkt wird.

Das natürliche Mikrobiom des Urogenitaltrakts bzw. die Vaginalflora können jedoch aus dem Gleichgewicht geraten bzw. gestört werden:
So führen beispielsweise Behandlungen mit Antibiotika nicht nur zu einer Abtötung der pathogenen Zielorganismen, sondern darüber hinaus auch zu einer Schädigung der Vaginalflora, da Antibiotika nicht spezifisch gegen einzelne Bakterienspezies gerichtet sind, sondern üblicherweise ein breites Wirkspektrum besitzen. Durch Behandlungen mit Antibiotika werden somit auch die Milchsäure produzierenden Bakterien der Vaginalflora abgetötet. Auch können hormonelle Veränderungen, wie sie beispielsweise mit Schwangerschaften, der Einnahme bzw. Verwendung von hormonhaltigen Verhütungsmitteln, der Menopause oder der Menstruation etc. einhergehen, zu einem Ungleichgewicht bzw. zu einer Störung der Vaginalflora führen. Weiterhin sind auch chronische Krankheiten, wie Diabetes, oder das insbesondere dauerhafte Tragen von Windeln bzw. Inkontinenzvorlagen insbesondere aufgrund von Harninkontinenz oftmals mit einer Fehlbesiedelung des Urogenitaltrakts verbunden.

Wird die Zusammensetzung der Vaginalflora gestört bzw. in ein Ungleichgewicht gebracht, erhöht sich das Risiko einer Kolonisierung des Urogenitaltrakts mit pathogenen Erregern. Kommt es zu einer Ausbreitung von pathogenen Keimen im Bereich des Urogenitaltrakts bzw. der Windelregion, können Erkrankungen, wie Vaginitis bzw. Vaginose oder eine so genannte Windeldermatitis bzw. ein Windelekzem, resultieren. Darüber hinaus können auch Harnwegsinfekte entstehen. Zu den an den vorgenannten Erkrankungen beteiligten Keimen zählen insbesondere *Escherichia coli* sowie Bakterien der Gattungen *Enterococcus, Pseudomonas, Proteus, Klebsiella, Streptococcus, Staphylococcus, Chlamydia, Trichomonas* oder *Gardnerella.* Darüber hinaus können auch Infektionen mit Pilzen, insbesondere der Gattung *Candida,* zu entzündlichen Beschwerden des Urogenitaltrakts führen, wenn die Vaginalflora in ihrer Zusammensetzung gestört ist.

Zwar existieren im Stand der Technik durchaus Ansätze, um derartigen Infektionen vorzubeugen bzw. diese zu behandeln, allerdings führen diese oftmals nicht zu dem gewünschten Erfolg.

Durch häufiges Waschen im Rahmen der normalen Körperhygiene kann zwar grundsätzlich Infektionen des Urogenitaltrakts vorgebeugt werden, allerdings werden durch den Einsatz der üblichen Seifen und Pflegeprodukte nicht nur pathogene Keime in ihrem Wachstum gehemmt bzw. abgetötet. Auch die notwendige und natürlich vorkommende Vaginalflora kann durch häufiges Waschen des Urogenitaltrakts weiterführend geschädigt werden. Sobald eine Kolonisierung der Vaginalflora mit pathogenen Keimen vorliegt, reicht die normale Körperhygiene nicht aus, um Infektionen zu behandeln bzw. die gesunde Vaginalflora wiederherzustellen.

Darüber hinaus existieren Ansätze, welche die Applikation von Milchsäurebakterien bzw. von in der natürlichen Vaginalflora vorkommenden Bakterien am insbesondere weiblichen Urogenitaltrakt vorsehen. So sind unter anderem Tabletten und Gele bekannt, welche zur vaginalen Anwendung vorgesehen sind und Milchsäurebakterien enthalten. Derartige Gele bzw. Tabletten sind jedoch in der Handhabung äußerst unkomfortabel. Auch ist eine längerfristige bzw. kontinuierliche Abgabe von Milchsäurebakterien an den Urogenitaltrakt auf diese Art und Weise nicht möglich.

Weiterhin wird zur Vorbeugung von Fehlbesiedelungen der Vaginalflora vorgeschlagen, Tampons vor ihrer Verwendung in Naturjoghurt zu tauchen. Die Verwendung von mit Naturjoghurt getränkten Tampons ist jedoch in mehrfacher Hinsicht nachteilig. Zum einen führt das häufige Tragen von Tampons auch außerhalb der Menstruation zu einem Austrocknen der Vaginalschleimhaut. Zum anderen entsprechen die in Naturjoghurt vorkommenden Milchsäurebakterien nicht denen, welche üblicherweise in der Vagnialflora vorliegen. Vielmehr kann Naturjoghurt sogar Milchsäurebakterien oder Hefen enthalten, welche zu einer Störung der Vaginalflora oder sogar zu Infektionen bzw. Entzündungen führen können.

Darüber hinaus sind im Stand der Technik probiotische Tampons bekannt, welche mit Milchsäurebakterien ausgerüstet sind. Problematisch an Tampons dieser Art ist jedoch, dass sie ebenfalls bei insbesondere längerfristiger Anwendung zu einer Austrocknung der Vaginalschleimhaut führen. Darüber hinaus werden die Milchsäurebakterien oftmals nicht in ausreichender Menge bzw. Effizienz aus den Tampons freigesetzt, so dass insgesamt keine gute Wirkeffizienz gewährleistet wird.

So betrifft die WO 2008/060200 A1 Sanitätsartikel, wie Damenbinden, Slipeinlagen, Inkontinenzprotektoren, Binden, Inkontinenzvorlagen oder Tampons, wobei die Sanitätsartikel eine obere Schicht mit einer Zusammensetzung zur Hemmung von Mikroorganismen aufweisen. Die Zusammensetzung basiert auf mindestens einem extrazellulären Produkt von mindestens einem probiotischen Bakterium, gegebenenfalls mindestens einem probiotischen Bakterium sowie mindestens einem Additiv in Form einer organischen Säure.

Die DE 2 309 575 betrifft zudem einen Vaginaltampon, welcher eine Imprägnierung auf Basis von im Scheidenmilieu physiologisch vorkommenden Substanzen aufweist, um den physiologischen Säureschutz aufrechtzuerhalten. Insbesondere kann es sich bei der Imprägnierung um Milchsäure oder alternativ Döderlein-Bakterien handeln.

Weiterhin betrifft die EP 1 880 727 A2 eine topische, vaginal zu applizierende pharmazeutische Zusammensetzung mit einem lokalen Effekt, welche Lactobacilli, Milchsäure und Krameria-Extrakt enthält.

In der wissenschaftlichen Publikation gemäß Haya et al: "Importance of Lactic Acid in Maintaining Vaginal Health: A review of Vaginitis and Vaginosis Etiopathogenic Bases and a Proposal for a new Treatment", erschienen in Open Journal of Obstetrics and Gynecology, 2014, 4, 787-799, wird zudem die Rolle bzw. der positive Einfluss der Applikation von Milchsäure bei der Behandlung von vaginalen Symptomen, insbesondere entzündlichen Erkrankungen, wie Vaginitis bzw. Vaginose, beschrieben.

Der vorliegenden Erfindung liegt daher die Aufgabe zugrunde, einen Hygieneartikel bereitzustellen, welcher sich zur prophylaktischen bzw. therapeutischen Behandlung von Erkrankungen des Urogenitaltrakts bzw. der Windelregion eignet, wobei die zuvor geschilderten Nachteile des Standes der Technik zumindest weitgehend vermieden oder aber wenigstens abgeschwächt werden sollen.

Insbesondere liegt der vorliegenden Erfindung die Aufgabe zugrunde, einen Hygieneartikel bereitzustellen, welcher sich durch eine hohe Wirkeffizienz in Bezug auf die Wiederherstellung einer gesunden bzw. natürlichen Vaginalflora auszeichnet und darüber hinaus auch komfortabel in der Handhabung ist.

Zur Lösung der zuvor geschilderten Aufgabe schlägt die vorliegende Erfindung einen Hygieneartikel gemäß Anspruch 1 vor; weitere vorteilhafte Ausgestaltungen sind Gegenstand der diesbezüglichen abhängigen Ansprüche.

Weiterer Gegenstand der vorliegenden Erfindung ist ein erfindungsgemäßer Hygieneartikel zur Verwendung gemäß dem diesbezüglich unabhängigen Anspruch.

Darüber hinaus ist Gegenstand der vorliegenden Erfindung ein Hygieneartikel nach der vorliegenden Erfindung zur Verwendung bei der prophylaktischen bzw. therapeutischen Behandlung von Erkrankungen des Urogenitaltrakts bzw. der Windelregion gemäß dem betreffenden unabhängigen Anspruch.

Ebenfalls ist Gegenstand der vorliegenden Erfindung die erfindungsgemäße Wirkstoffkombination zur Verwendung gemäß dem diesbezüglich unabhängigen Anspruch.

Weiterhin ist Gegenstand der vorliegenden Erfindung auch eine Kombination ("Wirkstoffkombination"), insbesondere zur Ausrüstung eines Hygieneartikels (Sanitärartikels) mit keimhemmenden, insbesondere bakterien- und/oder pilzhemmenden Eigenschaften gemäß dem betreffenden unabhängigen Anspruch.

Schließlich ist Gegenstand der vorliegenden Erfindung auch eine Kombination ("Wirkstoffkombination") zur Verwendung bei der prophylaktischen und/oder therapeutischen Behandlung von Erkrankungen des Urogenitaltrakts und/oder der Windelregion, insbesondere Vaginalinfektionen, Ureterinfektionen, Vulvitis, Windeldermatitis und/oder Ekzemen.

Weiterhin ist bei allen nachstehend genannten relativen bzw. prozentualen, insbesondere gewichtsbezogenen Mengenangaben zu beachten, dass diese im Rahmen der vorliegenden Erfindung vom Fachmann derart auszuwählen sind, dass in der Summe der jeweiligen Inhaltsstoffe, Wirkstoffe, Zusatz- bzw. Hilfsstoffe oder dergleichen stets 100 % bzw. 100 Gew.% resultieren. Dies versteht sich für den Fachmann aber von selbst.

Zudem gilt, dass alle im Folgenden genannten Parameterangaben oder dergleichen grundsätzlich mit genormten oder explizit angegebenen Bestimmungsverfahren oder aber mit dem Fachmann an sich geläufigen Bestimmungsmethoden bestimmt bzw. ermittelt werden können.

Dies vorausgeschickt, wird nun die vorliegende Erfindung nachfolgend im Detail erläutert.

Gegenstand der vorliegenden Erfindung - gemäß einem **ersten** Aspekt der vorliegenden Erfindung - ist somit ein Hygieneartikel, insbesondere zur Verwendung bei der prophylaktischen und/oder therapeutischen Behandlung von Erkrankungen des Urogenitaltrakts und/oder der Windelregion, insbesondere Vaginalinfektionen, Ureterinfektionen, Vulvitis, Windeldermatitis und/oder Ekzemen, in Form einer Damenbinde und/oder Slipeinlage,
wobei der Hygieneartikel eine Kombination ("Wirkstoffkombination") von
(a) Milchsäure produzierenden Bakterien, ausgewählt aus der Gruppe von *Lactobacillus acidophilus,* in Form von lyophilisierten und tyndallisierten Zellen ("Komponente (a)") einerseits und
(b) Milchsäure und/oder deren Salzen und/oder Estern ("Komponente (b)") in einer Menge im Bereich von 0,1 bis 1.000 mg, bezogen auf den Hygieneartikel, andererseits
aufweist.

Auf Basis der erfindungsgemäßen Wirkstoffkombination von Milchsäure produzierenden Bakterien einerseits und Milchsäure bzw. deren Salzen und/oder Estern andererseits in den erfindungsgemäßen Hygieneartikeln wird die natürliche Vaginalflora, welche - wie eingangs bereits geschildert - insbesondere auf Milchsäure produzierenden Bakterien basiert, unterstützt bzw. wiederhergestellt. Ohne sich hierbei auf diese Theorie beschränken zu wollen, wird durch die Milchsäure der pH-Wert im Bereich des Urogenitaltrakts bzw. im Bereich der Windelregion auf ein leicht saures Milieu eingestellt, was einer Besiedelung durch pathogene Keime, welche zu Infektionen bzw. Entzündungen führen können, entgegenwirkt.

Durch den zusätzlichen Einsatz von Milchsäure produzierenden Bakterien wird weiterführend einer Kolonisierung des äußeren Bereichs des Urogenitaltrakts mit üblicherweise nicht in der Vaginalflora vorkommenden Mikroorganismen, insbesondere pathogenen Keimen, entgegengewirkt. Ohne sich hierbei auf diese Theorie beschränken zu wollen, werden durch die erfindungsgemäße Wirkstoffkombination optimale Wachstumsbedingungen für natürlicherweise in der Vaginalflora vorkommende Bakterien gefördert, wohingegen für pathogene bzw. unerwünschte Mikroorganismen ungünstige Wachstumsbedingungen geschaffen werden.

Die vorliegende Erfindung ist mit zahleichen weiteren Vorteilen und Besonderheiten verbunden, welche nachfolgend in nicht beschränkender Weise diskutiert sind und als Indiz für Patentfähigkeit zu werten sind.

Im Rahmen der vorliegenden Erfindung wird somit ein Hygieneartikel, insbesondere in Form einer Damenbinde bzw. Slipeinlage, bereitgestellt, welcher Fehlbesiedelungen, insbesondere Infektionen, des Urogenitaltrakts bzw. der Windelregion, insbesondere bei Frauen, vorbeugen bzw. eindämmen kann. Insbesondere kann einer Kolonisierung mit pathogenen Erregern, wie Mikroorganismen der Gattungen *Escherichia, Staphylococcus, Candida, Chlamydia, Trichomonas* oder *Gardnerella,* vorgebeugt bzw. können bestehende Infektionen behandelt werden. Der erfindungsgemäße Hygieneartikel eignet sich somit insbesondere zur Behandlung einer gestörten Vaginalflora sowohl auf prophylaktischer als auch auf therapeutischer Ebene.

Der erfindungsgemäße Hygieneartikel zeichnet sich zudem durch seine hervorragende Verträglichkeit aus. Die erfindungsgemäß eingesetzte Wirkstoffkombination ist zumindest im Wesentlichen frei von Nebenwirkungen. Insbesondere kann auch ein Austrocknen der Schleimhäute verhindert werden, so dass die erfindungsgemäßen Hygieneartikel auch längerfristig verwendet werden können.

Darüber hinaus zeichnen sich die erfindungsgemäßen Hygieneartikel gegenüber den aus dem Stand der Technik bekannten Hygieneartikeln mit Milchsäurebakterien durch einen schnelleren Wirkeintritt aus, welcher sich insbesondere - ohne sich hierbei auf diese Theorie beschränken zu wollen - durch den zusätzlichen Einsatz von Milchsäure begründen lässt. Durch die Milchsäure wird der pH-Wert der Vaginalflora unmittelbar mit der Anwendung des Hygieneartikels auf ein natürliches Milieu bzw. einen leicht sauren pH-Wert reguliert, so dass auch unmittelbar mit der Verwendung des Hygieneartikels die Wachstumsbedingungen für die natürlicherweise in der Vaginalflora vorkommenden Bakterien verbessert werden bzw. ein sofortiger Wirkeintritt gewährleistet wird.

Weiterhin ist die Wirkdauer der Wirkstoffkombination des erfindungsgemäßen Hygieneartikels verlängert, da die Wirkstoffe über einen langen Zeitraum über die gesamte Anwendung des Hygieneartikels kontinuierlich aus dem Hygieneartikel an den insbesondere äußeren Urogenitaltrakt bzw. an die Windelregion abgegeben werden können.

Darüber hinaus zeichnet sich der erfindungsgemäße Hygieneartikel durch eine hervorragende Lagerstabilität aus. Insbesondere erfolgt keine unkontrollierte Vermehrung der in dem Hygieneartikel vorliegenden Milchsäure produzierenden Bakterien auch unter längerfristiger Lagerung. Durch den Einsatz der Milchsäure produzierenden Bakterien in Form von lyophilisierten und tyndallisierten Zellen kann die Lagerstabilität verbessert werden.

Weitere Vorteile, Merkmale, Eigenschaften und Aspekte ergeben sich aus der folgenden Beschreibung bevorzugter Ausführungsformen anhand der Zeichnungen. Es zeigt:
- Fig. 1: (nicht erfindungsgemäß) eine schematische Darstellung des erfindungsgemäßen Hygieneartikels auf Basis einer Draufsicht auf die im Tragezustand dem Körper abgewandten Seite, wobei der Hygieneartikel gemäß dieser bevorzugten Ausführungsform mehrlagig bzw. mehrschichtig ausgebildet ist; und
- Fig. 2: eine schematische Schnittdarstellung des erfindungsgemäßen Hygieneartikels, wobei der Hygieneartikel gemäß dieser bevorzugten Ausführungsform mehrlagig bzw. mehrschichtig ausgebildet ist.

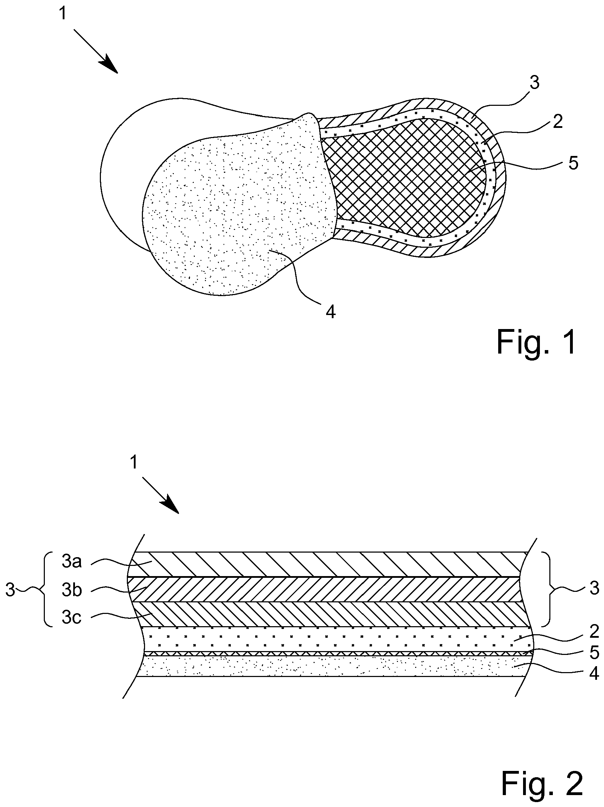

Fig. 1 zeigt einen Hygieneartikel (Sanitärartikel) 1 gemäß einer bevorzugten Ausführungsform (nicht erfindungsgemäß) welcher zur Verwendung bei der prophylaktischen und/oder therapeutischen Behandlung von Erkrankungen des Urogenitaltrakts und/oder der Windelregion, insbesondere Vaginalinfektionen, Ureterinfektionen, Vulvitis, Windeldermatitis und/oder Ekzemen, geeignet ist und in Form einer Damenbinde und/oder Slipeinlage vorliegt, wobei der Hygieneartikel eine Kombination ("Wirkstoffkombination") von (a) Milchsäure produzierende Bakterien, insbesondere der Gattung *Lactobacillus* oder *Bifidobacterium,* ("Komponente (a)") einerseits und (b) Milchsäure und/oder deren Salzen und/oder Estern ("Komponente (b)") andererseits aufweist.

Im Rahmen der vorliegenden Erfindung werden unter dem Begriff "Hygieneartikel", synonym auch als "Sanitätsartikel" bezeichnet, insbesondere Artikel bzw. Produkte verstanden, welche der Körperhygiene im Bereich des Urogenitaltrakts bzw. der Windelregion dienen. Insbesondere handelt es sich bei den Hygieneartikeln nach der vorliegenden Erfindung um Produkte für die Monatshygiene der Frau bzw. um Sanitätsartikel insbesondere zur Verwendung bei Harninkontinenz. Vorzugsweise handelt es sich bei den erfindungsgemäßen Hygieneartikeln um Damenbinden, Slipeinlagen, Windeln bzw. Windelhosen, bevorzugt Damenbinden und/oder Slipeinlagen.

Bei den Milchsäure produzierenden Bakterien handelt es sich um eine sehr heterogene Gruppe Gram-positiver anaerober, dabei aber im Allgemeinen aerotoleranter Bakterien, welche die Fähigkeit besitzen, Zucker zu Milchsäure zu vergären (was üblicherweise auch als Milchsäuregärung bezeichnet wird). Insbesondere gehören der Gruppe der Milchsäuregärung betreibenden Bakterien Spezies der Gattungen *Lactobacillus,* welche im Allgemeinen auch als Milchsäurebakterien bezeichnet werden, und *Bifidobacterium* an. In Bezug auf ihre morphologischen Eigenschaften sind Milchsäure produzierende Bakterien bzw. Milchsäurebakterien sehr heterogen und zeichnen sich vielmehr durch ihren spezifischen Stoffwechsel aus. Im Gegensatz zu den meisten anderen Bakterienarten sind Milchsäurebakterien zur Energiegewinnung auf Kohlenhydrate und zahlreiche Suppline angewiesen. Bezüglich des Stoffwechsels können Milchsäurebakterien in homofermentative Bakterien und heterofermentative Milchsäurebakterien unterteilt werden, wobei homofermentative Bakterien als Endprodukt ihres Gärungsstoffwechsels ausschließlich Milchsäure produzieren und heterofermentative Bakterien bzw. Bifidobakterien neben Milchsäure bzw. Laktat auch andere Endprodukte, insbesondere Acetat bzw. Essigsäure, Ethanol und CO₂, produzieren.

Da Milchsäurebakterien aufgrund ihres degenerierten bzw. eingeschränkten Stoffwechsels auf eine nährstoff- und supplinreiche Umgebung angewiesen sind, siedeln sie sich vor allem in Milch und Milchprodukten, im Darm bzw. in Schleimhäuten von Säugetieren sowie in lebenden oder sich zersetzenden Pflanzen an. Für den Menschen gehören die Milchsäurebakterien zu den wichtigsten und nützlichen Vertretern der menschlichen Darmflora, aber auch - wie zuvor bereits erwähnt - zu der Hautflora, insbesondere der Vaginalflora.

In Bezug auf den erfindungsgemäßen Hygieneartikel ist es vorgesehen, dass der Hygieneartikel die Milchsäure produzierenden Bakterien in Form von inaktiven Zellen, nämlich lyophilisierten und tyndallisierten Zellen, aufweist. Erfindungsgemäß liegen die Milchsäure produzierenden Bakterien in dem Hygieneartikel in Form von inaktiven Zellen, nämlich lyophilisierten und tyndallisierten Zellen, vor.

Unter lyophilisierten Zellen bzw. Bakterien werden im Rahmen der vorliegenden Erfindung insbesondere Bakterien verstanden, welche unter Sublimation mittels Lyophilisierung, synonym auch als Gefriertrocknung bezeichnet, schonend getrocknet und auf diese Weise lagerfähig bzw. haltbar gemacht werden. Bei tyndallisierten Zellen bzw. Bakterien handelt es sich im Rahmen der vorliegenden Erfindung um eine hitzeinaktivierte Form von Milchsäure produzierenden Bakterien. Im Rahmen der vorliegenden Erfindung werden für den Hygieneartikel Milchsäure produzierende Bakterien eingesetzt, welche zunächst mittels Tyndallisierung hitzeinaktiviert und anschließend mittels Lyophilisierung bzw. Gefriertrocknung haltbar gemacht werden. Tyndallisierte und anschließend lyophilisierte Zellen zeichnen sich dadurch aus, dass sie zwar nicht mehr lebensfähig und somit inaktiv sind, die Integrität der Zellwand bzw. die Zellstrukturen erhalten bleibt, so dass die Zellen weiterhin immunstimulierende Eigenschaften besitzen.

Der Vorteil des Einsatzes von tyndallisierten und lyophilisierten Bakterien für die erfindungsgemäßen Hygieneartikel besteht insbesondere darin, dass die Haltbarkeit bzw. Lagerstabilität der mit Milchsäure produzierenden Bakterien ausgerüsteten Hygieneartikel verbessert ist, da einer unkontrollierten Vermehrung der Milchsäure produzierenden Bakterien während der Lagerung vorgebeugt werden kann. Darüber hinaus war es vollkommen überraschend, dass auch mit inaktiven, insbesondere hitzeinaktivierten Milchsäure produzierenden Bakterien eine hervorragende Wirkeffizienz in Bezug auf die Wiederherstellung bzw. Behandlung der Vaginalflora erzielt wird. Dies kann - ohne sich hierbei auf diese Theorie beschränken zu wollen - zum einen auf eine physikalische Verdrängung unerwünschter Mikroorganismen, insbesondere pathogener Keime, durch die tyndallisierten und lyophilisierten Zellen und zum anderen auf die immunstimulierende Wirkung zurückgeführt werden.

Die erfindungsgemäß eingesetzten tyndallisierten und lyophilisierten Bakterien führen somit einerseits zu einer signifikant verbesserten Lagerfähigkeit bzw. Haltbarkeit der Hygieneartikel und andererseits zu einer hervorragenden Wirksamkeit in Bezug auf die Wiederherstellung einer gesunden Vaginalflora und auf die Prophylaxe bzw. Behandlung von Infektionen des Urogenitaltrakts.

Milchsäure produzierende Bakterien sind ausgewählt (nicht erfindungsgemäß) aus der Gruppe von Milchsäuregärung betreibenden Bakterien, insbesondere in der Normalflora der menschlichen Haut, Vagina und/oder Vulva vorkommende Milchsäuregärung betreibende Bakterien.

Milchsäure produzierende Bakterien sind ausgewählt (nicht erfindungsgemäß) aus der Gruppe von Bakterien der Gattung *Lactobacillus, Bifidobacterium* und/oder *Pediococcus,* vorzugsweise *Lactobacillus.*

Milchsäure produzierende Bakterien sind ausgewählt (nicht erfindungsgemäß) aus der Gruppe von *Bifidobacterium spp., Lactobacillus iners, Lactobacillus delbruekii, Lactobacillus crispatus, Lactobacillus vaginalis, Lactobacillus acidophilus, Lactobacillus jensenii, Lactobacillus buchneri und*/*oder Lactobacillus gasseri,* insbesondere *Lactobacillus acidophilus.*

Gemäß der vorliegenden Erfindung sind die Milchsäure produzierenden Bakterien ausgewählt aus der Gruppe von *Lactobacillus acidophilus* in Form von inaktiven Zellen, in Form von lyophilisierten und tyndallisierten Zellen.

Um eine gute Wirksamkeit der erfindungsgemäßen Hygieneartikel zu gewährleisten, kann es im Rahmen der vorliegenden Erfindung vorgesehen sein, dass der Hygieneartikel die Milchsäure produzierenden Bakterien in einer Menge im Bereich von 10¹ bis 10³⁰ KBE (koloniebildende Einheiten), insbesondere im Bereich von 10² bis 10²⁰ KBE, vorzugsweise im Bereich von 10⁵ bis 10¹⁸ KBE, bevorzugt im Bereich von 10¹⁰ bis 10¹⁵ KBE, bezogen auf den Hygieneartikel, aufweist.

Im Rahmen der vorliegenden Erfindung ist es vorgesehen, um eine gute Wirksamkeit der erfindungsgemäßen Hygieneartikel zu gewährleisten, dass der Hygieneartikel Milchsäure und/oder deren Salze und/oder Ester in einer Menge im Bereich von 0,1 bis 1.000 mg, bevorzugt im Bereich von 1 bis 500 mg, bezogen auf den Hygieneartikel, aufweist. Was den Begriff der koloniebildenden Einheiten (KBE), synonym in Englisch auch als *Colony Forming Unit* (CFU) bezeichnet, anbelangt, handelt es sich dabei um eine Größe, welche üblicherweise in der Mikrobiologie zur Quantifizierung von Mikroorganismen eingesetzt wird. Dabei wird die Lebendzellzahl von Mikroorganismen in einem Material mit Hilfe dem Fachmann an sich bekannter Kultivierungsmethoden bestimmt. Dabei wird meistens eine Probe des Materials, dessen Mikroorganismengehalt bestimmt werden soll, auf der Oberfläche eines Kulturmediums gleichmäßig und üblicherweise in starker Verdünnung verteilt, so dass die in der Probe enthaltenen Individuen der Mikroorganismen einzeln und voneinander beabstandet zum Liegen kommen und durch Wachstum und Vermehrung sichtbare Kolonien ausbilden. Über die Anzahl der Kolonien kann die Anzahl der ursprünglich in der Probe enthaltenen Individuen bestimmt werden. Im Fall von inaktiven Zellen bzw. Überdauerungsformen, wie sie im Rahmen der vorliegenden Erfindung eingesetzt werden können, insbesondere lyophilisierten bzw. tyndallierten Zellen, entspricht die Anzahl der koloniebildenden Einheiten der Zellzahl an inaktiven Zellen.

Was den strukturellen Aufbau des erfindungsgemäßen Hygieneartikels anbelangt, sind nachfolgend bevorzugte Ausführungsformen, welche jedoch nicht beschränkend zu verstehen sind, beschrieben:
Im Rahmen der vorliegenden Erfindung hat es sich als besonders vorteilhaft erwiesen, wenn der Hygieneartikel eine im Trage- bzw. Anwendungszustand dem Körper zugewandte erste Seite und eine im Trage- bzw. Anwendungszustand dem Körper abgewandte, insbesondere der ersten Seite gegenüberliegende zweite Seite aufweist. Dabei kann es vorgesehen sein, dass der Hygieneartikel die Kombination von Milchsäure produzierenden Bakterien und Milchsäure und/oder deren Salzen und/oder Estern auf der im Trage- bzw. Anwendungszustand dem Körper zugewandten ersten Seite aufweist. In diesem Zusammenhang kann es auch vorgesehen sein, dass der Hygieneartikel imstande ist, die Kombination von Milchsäure produzierenden Bakterien und Milchsäure und/oder deren Salzen und/oder Estern im Trage- bzw. Anwendungszustand an die im Trage- bzw. Anwendungszustand mit dem Hygieneartikel in Kontakt befindlichen Körperbereich, insbesondere an den Urogenitaltrakt und/oder die Windelregion, des Trägers oder Anwenders abzugeben.

Weitere insbesondere bevorzugte Ausführungsformen der vorliegenden Erfindung ergeben sich anhand der folgenden Beschreibung auf Basis der Zeichnungen:
Fig. 1 und Fig. 2 zeigen den Aufbau eines erfindungsgemäßen Hygieneartikels 1 in Form einer Slipeinlage bzw. Damenbinde, wobei Fig. 1 (nicht erfindungsgemäß) eine schematische Darstellung auf Basis einer Draufsicht auf die dem Körper abgewandte Seite des Hygieneartikels 1 in Form einer Slipeinlage und/oder Binde zeigt. Fig. 2 hingegen zeigt eine schematische Schnittdarstellung einer bevorzugten Ausführungsform des erfindungsgemäßen Hygieneartikels 1 in Form einer mehrschichtigen bzw. mehrlagigen Slipeinlage bzw. Damenbinde.

Die Darstellung des Hygieneartikels 1 in Form einer Slipeinlage bzw. Damenbinde in den Figuren ist keinesfalls beschränkend zu verstehen. Vielmehr versteht es sich für den Fachmann von selbst, dass die veranschaulichten Merkmale auch ohne Weiteres auf andere Hygieneartikel, wie Windeln, Windelhosen oder Inkontinenzvorlagen, übertragen werden können.

Gemäß einer bevorzugten Ausführungsform der vorliegenden Erfindung kann es vorgesehen sein, dass der Hygieneartikel 1 schichtartig aufgebaut ist. Insbesondere kann es vorgesehen sein, dass der Hygieneartikel 1 mehrere Schichten aufweist.

Im Zusammenhang mit seinem Aufbau kann der Hygieneartikel 1 erfindungsgemäß eine vorzugsweise flüssigkeitsundurchlässige Rückenschicht 2 aufweisen. Weiterhin kann es diesbezüglich vorgesehen sein, dass die Rückenschicht 2 die im Trage- bzw. Anwendungszustand dem Körper abgewandte Seite des Hygieneartikels 1 darstellt. Was darüber hinaus die spezielle Ausgestaltung der Rückenschicht 2 anbelangt, so kann es vorgesehen sein, dass das Material der Rückenschicht 2 aus Vliesen und/oder Folien, insbesondere auf Basis von Polyethylenen, Polypropylenen, Polyvinylidenchloriden, Polyestern, Polystyrolen, Polyurethanen, Polyamiden, natürlichen und/oder synthetischen Kautschuken sowie deren Kombinationen, ausgewählt ist.

Durch den Einsatz einer flüssigkeitsundurchlässigen Rückenschicht kann insbesondere ein Herauslaufen der von dem Hygieneartikel aufgenommenen Körperflüssigkeiten, insbesondere Blut und/oder Urin, aus dem erfindungsgemäßen Hygieneartikel verhindert werden, so dass die Kleidung vor Verschmutzungen geschützt wird.

Um darüber hinaus eine stabile Fixierung bzw. Anheftung des Hygieneartikels 1 an Wäschestücken, insbesondere Unterwäsche, zu ermöglichen, kann es vorgesehen sein, dass die Rückenschicht 2 mit einer Haftklebeschicht 5 ausgerüstet ist, wobei die Haftklebeschicht 5 ein Haft- und/oder Klebemittel enthalten bzw. hieraus bestehen kann. Was darüber hinaus die Auswahl des Haftklebemittels anbelangt, so wird eine gute Anheftung bzw. werden gute Klebeeigenschaften erzielt, wenn das Haftklebemittel aus der Gruppe von Polyurethanen, Polyvinylethern, Polyisobutylenen, Silikonen, (Meth-)Acrylaten, Polyethylenen, Polypropylenen, Polyvinylchloriden, Polyestern, Polystyrolen sowie deren Mischungen ausgewählt ist.

Weiterhin hat es sich in Bezug auf den erfindungsgemäßen Hygieneartikel als vorteilhaft erwiesen, wenn dieser eine abziehbare bzw. folienbasierte Schutzschicht 4 aufweist. In diesem Zusammenhang kann es insbesondere vorgesehen sein, dass die Schutzschicht 4 auf der Rückenschicht 2 des Hygieneartikels 1, insbesondere auf der im Trage- bzw. Anwendungszustand dem Körper abgewandten Seite der Rückenschicht 2, vorzugsweise auf der mit einer Haftklebeschicht 5 ausgerüsteten Seite der Rückenschicht 2, aufgebracht oder angeordnet ist. Darüber hinaus kann es in Bezug auf die Schutzschicht 4 vorgesehen sein, dass deren Material ausgewählt ist aus der Gruppe von Polyestern, Polyethylenen, Polypropylenen, Polyvinylchloriden, Aluminium, Papier, insbesondere mit einer Silikon-, Polyethylen-, Fluorsilikon- und/oder Polytetrafluorethylen-Beschichtung versehenem Papier, sowie deren Mischungen. Üblicherweise wird die abziehbare Schutzschicht 4 vor Gebrauch entfernt, so dass die sich darunter befindende Haftklebeschicht freigelegt wird und der Hygieneartikel 1 leicht an der Wäsche fixiert werden kann.

Um darüber hinaus eine gute Aufnahme von Körperflüssigkeiten, insbesondere Urin und/oder Blut, durch den erfindungsgemäßen Hygieneartikel 1 zu ermöglichen, kann es gemäß einer bevorzugten Ausführungsform der vorliegenden Erfindung vorgesehen sein, dass der Hygieneartikel 1 eine vorzugsweise flüssigkeitsdurchlässige, insbesondere saugfähige- und/oder absorptionsfähige Absorptionsschicht 3 aufweist. In diesem Zusammenhang kann es insbesondere vorgesehen sein, dass die Absorptionsschicht 3 die im Trage- bzw. Anwendungszustand dem Körper zugewandte Seite des Hygieneartikels 1 darstellt.

Gemäß einer besonders bevorzugten Ausführungsform der vorliegenden Erfindung, insbesondere wie sie in Fig. 2 dargestellt ist, kann es vorgesehen sein, dass die Absorptionsschicht 3 mehrlagig (mehrschichtig), insbesondere mindestens zweilagig, vorzugsweise mindestens dreilagig, ausgebildet ist.

Im Zusammenhang mit einer mehrlagigen bzw. mehrschichtigen Absorptionsschicht 3 hat es sich als besonders vorteilhaft erwiesen, wenn die mehrlagig ausgebildete Absorptionsschicht 3 eine vorzugsweise flüssigkeitsdurchlässige und/oder saugfähige, insbesondere an die der Schutzschicht 4 abgewandten Seite der Rückenschicht 2 des Hygieneartikels 1 angrenzende Kernschicht 3c aufweist. Darüber hinaus kann es vorgesehen sein, dass die mehrlagige bzw. mehrschichtige Absorptionsschicht eine vorzugsweise flüssigkeitsdurchlässige, insbesondere an die von der Rückenschicht 2 abgewandten Seite der Kernschicht 3c angrenzende Transferschicht 3b aufweist. Darüber hinaus kann es in diesem Zusammenhang vorgesehen sein, dass die mehrlagige bzw. mehrschichtige Absorptionsschicht 3 eine vorzugsweise flüssigkeitsdurchlässige, insbesondere an die von der Kernschicht 3c abgewandten Seite der Transferschicht 3b angrenzende Deckschicht 3a aufweist.

Die Kernschicht 3c der mehrlagigen Absorptionsschicht 3 kann dabei insbesondere zum Speichern bzw. zum Aufnehmen größerer Mengen der zu absorbierenden Körperflüssigkeiten, insbesondere Blut und/oder Urin, dienen. Durch die Transferschicht 3b erfolgt üblicherweise eine Verteilung der durch die Deckschicht 3a aufgenommenen Körperflüssigkeiten auf der Kernschicht 3c, damit die Kernschicht 3c über ihre gesamte Ausdehnung gleichmäßig die Körperflüssigkeiten aufnehmen kann, was insgesamt zu einer gesteigerten Saugeffizienz führt. Bei der Deckschicht 3a handelt es sich um die an die Haut bzw. Schleimhaut anliegende Außenschicht auf der im Tragezustand des Hygieneartikels 1 dem Körper zugewandten Seite des Hygieneartikels 1.

Im Hinblick auf die Kernschicht 3c hat es sich als besonders vorteilhaft erwiesen, wenn diese in Form eines Saugkerns ausgebildet ist. Insbesondere kann es in diesem Zusammenhang vorgesehen sein, dass die Kernschicht 3c auf Basis eines Superabsorbers ausgebildet ist bzw. dass die Kernschicht 3c ein superabsorbierendes Material enthält.

Im Rahmen der vorliegenden Erfindung werden unter einem Superabsorber bzw. einem superabsorbierenden Material insbesondere superabsorbierende Polymere verstanden, vorzugsweise Kunststoffe, welche in der Lage sind, ein Vielfaches ihres Eigengewichts an insbesondere polaren Flüssigkeiten, wie z.B. Wasser, Blut oder wässrigen Lösungen, aufzusaugen. Durch die Aufnahme von Flüssigkeiten quellen Superabsorber auf und bilden dabei Hydrogele aus. Grundsätzlich ist dem Fachmann der Einsatz von Superabsorbern in Hygieneartikeln bekannt, so dass bezüglich der Auswahl konkreter superabsorbierender Materialien keine weiteren Ausführungen an dieser Stelle notwendig sind. Nichtbeschränkende Beispiele für Superabsorber sind Polymere oder Copolymere auf Basis von (Meth-)Acrylsäure bzw. (Meth-)Acrylaten.

Je nach Einsatzzweck bzw. je nach aufzunehmender Menge an Flüssigkeiten kann darüber hinaus die Dicke der Kernschicht 3c zielgerichtet eingestellt werden. Für eine gute Saugfähigkeit bzw. Absorptionsfähigkeit hat es sich als besonders vorteilhaft erwiesen, wenn die Kernschicht 3c eine Dicke im Bereich von 0,001 bis 100 mm, insbesondere im Bereich von 0,01 bis 50 mm, vorzugsweise im Bereich 0,1 bis 30 mm, bevorzugt im Bereich von 0,5 bis 20 mm, besonders bevorzugt im Bereich von 1 bis 10 mm, aufweist.

Was darüber hinaus die Transferschicht 3b anbelangt, so hat es sich als vorteilhaft erwiesen, wenn im Rahmen der vorliegenden Erfindung das Material der Transferschicht 3b aus vorzugsweise textilen Flächenmaterialien, insbesondere Vliesen, Geweben, Gewirken, Gestricken, Papier und Watte, vorzugsweise Vliesen, ausgewählt ist. Auf Basis der vorgenannten Materialien, insbesondere Vliesen, kann aufgrund der Materialeigenschaften eine besonders gute Verteilung der aufgenommenen Körperflüssigkeiten auf der Kernschicht 3c, insbesondere in Form eines Saugkerns, erfolgen.

In Bezug auf die Deckschicht 3a kann es zudem vorgesehen sein, dass das Material der Deckschicht 3a aus vorzugsweise textilen Flächenmaterialien, insbesondere Vliesen, Folien, Geweben, Gewirken, Gestricken, Papier und Watte, vorzugsweise Vliesen und/oder Folien, ausgewählt ist. Die Deckschicht 3a sollte sich insbesondere durch eine gute Hautfreundlichkeit auszeichnen und darüber hinaus flüssigkeitsdurchlässig sein, so dass die zu absorbierenden Körperflüssigkeiten in das Innere des Hygieneartikels, insbesondere der Slipeinlage und/oder der Binde, gelangen können. Besonders gute Ergebnisse werden erzielt, wenn die Deckschicht 3a auf Basis von Vliesen oder perforierten Folien ausgebildet ist.

Um darüber hinaus eine gute Stabilität des erfindungsgemäßen Hygieneartikels 1 insbesondere im Anwendungszustand zu erlauben, kann es im Rahmen der vorliegenden Erfindung vorgesehen sein, dass die Schichten und/oder Lagen des Hygieneartikels 1 dauerhaft miteinander verbunden, bevorzugt miteinander verklebt, sind.

Was die Einbringung der erfindungsgemäßen Wirkstoffkombination in den Hygieneartikel 1 anbelangt, so ist diese variabel. Bevorzugte, jedoch nichtbeschränkende bzw. rein beispielhafte Ausführungsformen sind nachfolgend geschildert.

Gemäß einer bevorzugten Ausführungsform der vorliegenden Erfindung kann es vorgesehen sein, dass die Wirkstoffkombination der Komponenten (a) und (b) auf den Hygieneartikel 1 aufgebracht und/oder in den Hygieneartikel 1 eingebracht ist.

In diesem Zusammenhang hat es sich als besonders vorteilhaft erwiesen, wenn die Wirkstoffkombination der Komponenten (a) und (b) auf die Absorptionsschicht 3 aufgebracht und/oder in die Absorptionsschicht 3 eingebracht, bevorzugt in die Absorptionsschicht 3 eingebracht, ist. Das Auf- bzw. Einbringen kann insbesondere mittels Tränken und/oder Sprühen erfolgen. Darüber hinaus kann es im Zusammenhang mit der Einbringung der erfindungsgemäßen Wirkstoffkombination zudem vorgesehen sein, dass die Kombination der Komponenten (a) und (b) kontinuierlich oder diskontinuierlich, vorzugsweise diskontinuierlich, bevorzugt netzförmig, auf die Absorptionsschicht 3 aufgebracht und/oder in die Absorptionsschicht 3 eingebracht, bevorzugt in die Absorptionsschicht 3 eingebracht, ist.

Auf dieser Basis wird eine hervorragende Abgabe- bzw. Freisetzung der Milchsäure produzierenden Bakterien und der Milchsäure gewährleistet, ohne jedoch die Saugeffizienz bzw. die Absorptionseffizienz des erfindungsgemäßen Hygieneartikels zu beeinträchtigen.

Weiterhin kann es vorgesehen sein, dass die Kombination der Komponenten (a) und (b) auf die Deckschicht 3a aufgebracht und/oder in die Deckschicht 3a eingebracht ist. Weiterhin kann die erfindungsgemäße Wirkstoffkombination zwischen Deckschicht 3a und Transferschicht 3b eingebracht sein. Auch ist es möglich, dass die Komponenten (a) und (b) zwischen Transferschicht 3b und Kernschicht 3c eingebracht sind und/oder in Deckschicht 3a und Transferschicht 3b eingebracht sind.

Die erfindungsgemäß bevorzugte und zuvor geschilderte Einbringung der Komponenten (a) und (b) in den Hygieneartikel nach der vorliegenden Erfindung erlaubt eine langanhaltende und kontinuierliche Abgabe dieser Wirkstoffkombination an den Urogenitaltrakt, insbesondere die Vaginalflora, so dass die erfindungsgemäßen Hygieneartikel insgesamt eine verbesserte Wirkeffizienz im Vergleich zu den aus dem Stand der Technik bekannten Produkten zur Verbesserung der Vaginalflora aufweisen.

Um die Wirksamkeit bzw. Freisetzung der erfindungsgemäßen Wirkstoffkombination weiterführend zu verbessern, kann es gemäß einer besonders bevorzugten Ausführungsform der vorliegenden Erfindung vorgesehen sein, dass die Kombination der Komponenten (a) und (b) in einer Zusammensetzung vorliegt, wobei die Zusammensetzung außerdem mindestens einen physiologisch kompatiblen Träger (Exzipienten) aufweist. Durch die Inkorporierung der erfindungsgemäß eingesetzten Wirkkomponenten (a) und (b) werden die Inhaltsstoffe in eine Art Matrix eingebracht, so dass die kontinuierliche Freisetzung der Wirkkomponenten noch weiter verbessert werden kann.

Grundsätzlich kann die eingesetzte Menge an Milchsäure bzw. deren Salzen und/oder Estern in weiten Bereichen variieren. Besonders gute Ergebnisse im Hinblick auf Wirkeffizienz und Verträglichkeit der erfindungsgemäßen Hygieneartikel werden jedoch erzielt, wenn die Zusammensetzung Milchsäure und/oder deren Salze und/oder Ester in einer Menge im Bereich von 0,01 bis 15 Gew.-%, insbesondere im Bereich von 0,1 bis 10 Gew.-%, vorzugsweise im Bereich von 1 bis 5 Gew.-%, bevorzugt im Bereich von 2 bis 3 Gew.-%, bezogen auf die Zusammensetzung, enthält.

Auch die eingesetzte Menge an Milchsäure produzierenden Bakterien ist grundsätzlich variabel. Erfindungsgemäß weist die Zusammensetzung die Milchsäure produzierenden Bakterien in lyophilisierter und tyndallisierter Form in einer Menge im Bereich von 0,001 bis 500 mg/g Zusammensetzung, insbesondere im Bereich von 0,005 bis 100 mg/g Zusammensetzung, vorzugsweise im Bereich von 0,01 bis 50 mg/g Zusammensetzung, bevorzugt im Bereich von 0,1 bis 10 mg/g Zusammensetzung, besonders bevorzugt im Bereich von 0,5 bis 5 mg/g Zusammensetzung, ganz besonders bevorzugt im Bereich von 0,8 bis 1,5 mg/g Zusammensetzung, auf.

Gleichermaßen kann es vorgesehen sein, dass die Zusammensetzung pro g Zusammensetzung 10¹ bis 10³⁰ Zellen der Milchsäure produzierenden Bakterien, insbesondere 10² bis 10²⁰ Zellen der Milchsäure produzierenden Bakterien, vorzugsweise 10³ bis 10¹⁵ Zellen der Milchsäure produzierenden Bakterien, bevorzugt 10⁵ bis 10¹⁰ Zellen der Milchsäure produzierenden Bakterien, aufweist.

Darüber hinaus kann es im Rahmen der vorliegenden Erfindung vorgesehen sein, dass die Zusammensetzung gereinigtes Wasser in einer Menge im Bereich von 1 bis 99 Gew.-%, insbesondere im Bereich von 5 bis 90 Gew.-%, vorzugsweise im Bereich von 10 bis 80 Gew.-%, bevorzugt im Bereich von 15 bis 70 Gew.-%, besonders bevorzugt im Bereich von 20 bis 65 Gew.-%, ganz besonders bevorzugt im Bereich von 30 bis 60 Gew.-%, bezogen auf die Zusammensetzung, aufweist. Gleichermaßen kann es vorgesehen sein, dass die Zusammensetzung gereinigtes Wasser in einer Menge von höchstens 90 Gew.-%, vorzugsweise höchstens 80 Gew.-%, bevorzugt höchstens 70 Gew.-%, besonders bevorzugt höchstens 60 Gew.-%, bezogen auf die Zusammensetzung, aufweist.

Was darüber hinaus den Träger in der Zusammensetzung im Speziellen anbelangt, so hat es sich als vorteilhaft erwiesen, wenn dieser aus der Gruppe von Wasser, Ethanol, Isopropanol, Ethylcarbonat, Ethylacetat, Benzylalkohol, Benzylbenzoat, Propylenglycol, 1,3-Butylglycol, Wollfett, Baumwollsaatöl, Vaseline, Glycerinfettsäureestern, Polyethylenglykolen, Paraffinen sowie deren Mischungen und Kombinationen ausgewählt ist.

Die eingesetzte Menge des Trägers kann in weiten Bereichen variieren. Besonders gute Ergebnisse werden erzielt, wenn die Zusammensetzung den Träger in einer Menge im Bereich von 0,1 bis 50 Gew.-%, insbesondere im Bereich von 1 bis 40 Gew.-%, vorzugsweise im Bereich von 5 bis 30 Gew.-%, bevorzugt im Bereich von 10 bis 20 Gew.-%, bezogen auf die Zusammensetzung, enthält.

Um darüber hinaus die Eigenschaften der die Wirkstoffkombination enthaltenden Zusammensetzung weiterführend zu verbessern, kann es zudem vorgesehen sein, dass die Zusammensetzung außerdem mindestens einen Emulgator enthält.

Gemäß einer bevorzugten Ausführungsform ist der Emulgator aus der Gruppe von Fettalkoholen oder deren Estern mit Fettsäuren ausgewählt, insbesondere geradkettigen gesättigten Fettalkoholen mit 6 bis 30 Kohlenstoffatomen, vorzugsweise mit 8 bis 22 Kohlenstoffatomen, bevorzugt Laurylalkohol, Cetylalkohol, Stearylalkohol, Lignocerylalkohol, Cetylstearylalkohol oder Myristylalkohol, insbesondere Cetylstearylalkohol.

Was die Menge des mindestens einen Emulgators anbelangt, so kann diese in der Zusammensetzung im Bereich von 0,1 bis 20 Gew.-%, insbesondere im Bereich von 1 bis 15 Gew.-%, vorzugsweise im Bereich von 5 bis 12 Gew.-%, bevorzugt im Bereich von 7 bis 10 Gew.-%, bezogen auf die Zusammensetzung, variieren.

Im Hinblick auf die Wirkeffizienz des erfindungsgemäßen Hygieneartikels bzw. der Zusammensetzung, welche die erfindungsgemäße Wirkstoffkombination enthält, hat es sich als vorteilhaft erwiesen, wenn die Zusammensetzung einen pH-Wert im Bereich von 4 bis 8, insbesondere im Bereich von 4,5 bis 7, vorzugsweise im Bereich von 5 bis 6, bevorzugt im Bereich von 5,3 bis 5,8, aufweist. Im Rahmen der vorliegenden Erfindung ist es somit bevorzugt, dass die Zusammensetzung auf einen pH-Wert eingestellt wird, welcher im Wesentlichen dem pH-Wert der natürlichen Hautflora, insbesondere Vaginalflora, entspricht. Wie eingangs bereits geschildert, werden durch einen pH-Wert im sauren Bereich insbesondere pathogene Keime, insbesondere pathogene Bakterien und/oder Pilze, in ihrem Wachstum gehemmt, so dass eine Kolonisierung des Urogenitaltrakts bzw. der Windelregion mit pathogenen Erregern verhindert werden kann.

Darüber hinaus kann es vorgesehen sein, dass die Zusammensetzung mindestens einen Zusatz- und/oder Hilfsstoff aufweist, insbesondere ausgewählt aus der Gruppe von Verarbeitungshilfsmitteln, Stabilisatoren, Emulgatoren, Antioxidantien, Konservierungsstoffen, Feuchthaltemitteln, pH-Stell-mitteln, pH-Puffersubstanzen, Verdickungsmitteln, Färbe-, Puffer-, Riech-, Duft-, Streck-, Binde-, Netz- und/oder Konservierungsstoffen sowie deren Kombinationen.

Zudem ist es im Hinblick auf die Handhabbarkeit sowie die Freisetzung der erfindungsgemäßen Wirkstoffkombination vorteilhaft, wenn die Zusammensetzung in viskoser und/oder pastöser Form vorliegt bzw. wenn die Zusammensetzung als Salbe, Creme, Paste, Gel oder dergleichen vorliegt. Ohne sich hierbei auf diese Theorie beschränken zu wollen, wird durch die Inkorporierung der erfindungsgemäßen Wirkstoffkombination in eine viskose bzw. pastöse Zusammensetzung eine Matrix um die Inhaltsstoffe ausgebildet, so dass diese über einen längeren Zeitraum aus dem Hygieneartikel an die Haut bzw. Schleimhaut, insbesondere im Bereich des äußeren Urogenitaltrakts, abgegeben werden können.

Was die eingesetzte Menge der Zusammensetzung in dem erfindungsgemäßen Hygieneartikel anbelangt, so kann diese in weiten Bereichen variieren bzw. individuell auf die erforderliche Menge bzw. die Größe des Hygieneartikels eingestellt werden. Insbesondere kann es vorgesehen sein, dass der erfindungsgemäße Hygieneartikel die Zusammensetzung in einer Menge im Bereich von 0,01 bis 10 g, insbesondere im Bereich von 0,05 bis 8 g, vorzugsweise im Bereich von 0,1 bis 5 g, aufweist.

Insgesamt wird somit im Rahmen der vorliegenden Erfindung erstmals ein Konzept für einen Hygieneartikel bereitgestellt, welcher sich durch eine hervorragende Verträglichkeit, eine gute Handhabbarkeit und darüber hinaus durch eine hervorragende Wirkeffizienz im Zusammenhang mit der prophylaktischen bzw. therapeutischen Behandlung von Erkrankungen des Urogenitaltrakts bzw. der Windelregion zeigt, insbesondere im Hinblick auf eine Vorbeugung von Infektionen mit pathogenen Mikroorganismen, zum Beispiel Pilzinfektionen. Insbesondere kann durch den erfindungsgemäßen Hygieneartikel eine gesunde Vaginalflora unterstützt bzw. wiederhergestellt werden.

Wiederum weiterer Gegenstand der vorliegenden Erfindung - gemäß einem zweiten Aspekt der vorliegenden Erfindung - ist ein Hygieneartikel, wie er zuvor beschrieben wurde, zur Verwendung bei der prophylaktischen und/oder therapeutischen Behandlung von Erkrankungen des Urogenitaltrakts und/oder der Windelregion, insbesondere von Vaginalinfektionen, Ureterinfektionen, Vulvitis, Windeldermatitis und/oder Ekzemen.

Für weitergehende Einzelheiten zu diesem erfindungsgemäßen Aspekt kann auf die vorangehenden Ausführungen zu den übrigen Erfindungsaspekten verwiesen werden, welche diesbezüglich entsprechend gelten.

Des Weiteren ist Gegenstand der vorliegenden Erfindung - gemäß einem dritten Aspekt der vorliegenden Erfindung - die Verwendung einer Kombination ("Wirkstoffkombination") von
(a) Milchsäure produzierende Bakterien, ausgewählt aus der Gruppe von *Lactobacillus acidophilus,* in Form von lyophilisierten und tyndallisierten Zellen ("Komponente (a)") einerseits und
(b) Milchsäure und/oder deren Salzen und/oder Estern ("Komponente (b)") in einer Menge im Bereich von 0,1 bis 1.000 mg, bezogen auf den Hygieneartikel, andererseits
zur Ausrüstung eines Hygieneartikels, insbesondere eines Hygieneartikels, wie er zuvor beschrieben wurde, in Form einer Damenbinde und/oder Slipeinlage, mit keimhemmenden Eigenschaften, insbesondere eines Hygieneartikels zur Verwendung bei der prophylaktischen und/oder therapeutischen Behandlung von Erkrankungen des Urogenitaltrakts und/oder der Windelregion, insbesondere Vaginalinfektionen, Ureterinfektionen, Vulvitis, Windeldermatitis und/oder Ekzemen.

Für weitergehende Einzelheiten zu diesem erfindungsgemäßen Aspekt kann auf die vorangehenden Ausführungen zu den übrigen Erfindungsaspekten verwiesen werden, welche diesbezüglich entsprechend gelten.

Weiterhin ist Gegenstand der vorliegenden Erfindung - gemäß einem vierten Aspekt der vorliegenden Erfindung - eine Kombination ("Wirkstoffkombination") zur Ausrüstung eines Hygieneartikels, insbesondere wie er zuvor beschrieben wurde, mit keimhemmenden Eigenschaften, wobei die Kombination, insbesondere in jeweils (therapeutisch) wirksamen Mengen und/oder insbesondere zusammen mit einem kompatiblen Träger,
(a) Milchsäure produzierende Bakterien, ausgewählt aus der Gruppe von *Lactobacillus acidophilus,* in Form von lyophilisierten und tyndallisierten Zellen ("Komponente (a)") einerseits und
(b) Milchsäure und/oder deren Salze und/oder Ester in einer Menge im Bereich von 0,1 bis 1.000 mg, bezogen auf den Hygieneartikel, ("Komponente (b)") andererseits
enthält.

Für weitergehende Einzelheiten zu diesem erfindungsgemäßen Aspekt kann auf die vorangehenden Ausführungen zu den übrigen Erfindungsaspekten verwiesen werden, welche diesbezüglich entsprechend gelten.

Schließlich ist Gegenstand der vorliegenden Erfindung - gemäß einem fünften Aspekt der vorliegenden Erfindung - eine Kombination ("Wirkstoffkombination") zur Verwendung bei der prophylaktischen und/oder therapeutischen Behandlung von Erkrankungen des Urogenitaltrakts und/oder der Windelregion, insbesondere Vaginalinfektionen, Ureterinfektionen, Vulvitis, Windeldermatitis und/oder Ekzemen,
wobei die Kombination, insbesondere in jeweils (therapeutisch) wirksamen Mengen und/oder insbesondere zusammen mit einem kompatiblen Träger,
(a) Milchsäure produzierende Bakterien, ausgewählt aus der Gruppe von *Lactobacillus acidophilus,* in Form von lyophilisierten und tyndallisierten Zellen ("Komponente (a)") einerseits und
(b) Milchsäure und/oder deren Salze und/oder Ester in einer Menge im Bereich von 0,1 bis 1.000 mg, bezogen auf den Hygieneartikel, ("Komponente (b)") andererseits
enthält.

Für weitergehende Einzelheiten zu diesem erfindungsgemäßen Aspekt kann auf die vorangehenden Ausführungen zu den übrigen Erfindungsaspekten verwiesen werden, welche diesbezüglich entsprechend gelten.

Die folgenden Ausführungsbeispiele dienen lediglich der Veranschaulichung der vorliegenden Erfindung, ohne die vorliegende Erfindung jedoch hierauf zu beschränken.

### Ausführungsbeispiele:

### I. Bereitstellung von erfindungsgemäßen Hygieneartikeln und von Vergleichshygieneartikeln

Um die Hygieneartikel nach der vorliegenden Erfindung in Bezug auf ihre Wirksamkeit, ihre Wirkeffizienz sowie ihre Verträglichkeit zu untersuchen, werden erfindungsgemäße Hygieneartikel sowie Vergleichshygieneartikel bereitgestellt. Die Hygieneartikel werden zur Behandlung von geschädigter bzw. gestörter Vaginalflora eingesetzt. Anschließend wird der Behandlungserfolg überprüft und die Hygieneartikel werden in Bezug auf ihre Wirkeffizienz miteinander verglichen.

Bei den erfindungsgemäßen Hygieneartikeln und den Vergleichshygieneartikeln handelt es sich um Slipeinlagen, welche eine Rückenschicht auf Basis einer wasserundurchlässigen bzw. flüssigkeitsundurchlässigen Wäscheschutzfolie, einen Saugkern auf Basis eines Superabsorbers, eine Transferlage sowie eine Deckschicht auf Basis eines hautverträglichen Materials aufweisen. Darüber hinaus ist die Rückenschicht der Slipeinlagen mit einem Haftkleber und einer abziehbaren Schutzschicht ausgerüstet, so dass die Slipeinlagen problemlos an der Unterwäsche der Träger(innen) befestigt werden können.

Weiterhin sind die Slipeinlagen unter der Deckschicht durch netzförmigen Auftrag mit wirkstoffhaltigen Zusammensetzungen bzw. mit Placebo-Zusammensetzungen (d.h. wirkstofffreien Zusammensetzungen) versehen. Bei den Zusammensetzungen handelt es sich um hautaffine Cremezusammensetzungen in Form von wasserhaltigen hydrophilen Salben mit einem pharmazeutischen Trägermaterial auf Basis einer Kombination von Propylenglykol, Vaseline und Paraffin. Darüber hinaus enthalten die Zusammensetzungen als Emulgator Cetylstearylalkohol und gereinigtes Wasser.

Die erfindungsgemäßen **Slipeinlagen A** enthalten in der Zusammensetzung zudem 2,5 Gew.-% Milchsäure in Form von Natrium-Lactat und darüber hinaus 1 mg/g Zusammensetzung tyndallisierte Zellen von *Lactobacillus acidophilus,* jeweils bezogen auf die Zusammensetzung. Die **Vergleichsslipeinlage B** enthält in der Zusammensetzung 2,5 Gew.-% Milchsäure in Form von Natrium-Lactat, bezogen auf die Zusammensetzung, jedoch keine Milchsäure produzierenden Bakterien. Darüber hinaus enthält die **Vergleichsslipeinlage** C in der Zusammensetzung 1 mg/g tyndallisierte Zellen von *Lactobacillus acidophilus,* bezogen auf die Zusammensetzung; Slipeinlage C ist jedoch frei von Milchsäure. Schließlich weist eine weitere **Vergleichsslipeinlage** D eine Zusammensetzung auf, welche auf einer wasserhaltigen hydrophilen Salbe ohne Milchsäure und ohne Milchsäure produzierende Bakterien aufweist.

### II. Wirksamkeitsstudien zur Behandlung von gestörter Vaginalflora

Im Rahmen einer Wirksamkeitsstudie werden insgesamt 80 Probandinnen im Alter von 18 bis 45 Jahren herangezogen, welche unter einer Fehlbesiedelung des Urogenitaltrakts bzw. der Vaginalflora leiden. Insbesondere sind die Probandinnen über einen Zeitraum von mehr als einem Jahr regelmäßig an rezidivierenden Infektionen, insbesondere mit Pilzen der Gattung *Candida* sowie den Bakterien *Gardnerella vaginalis, Trichomonas vaginalis* und/oder *Atopobium vaginae,* betroffen. Die Probandinnen werden in vier Untersuchungsgruppen A bis D von jeweils 20 Probandinnen unterteilt und mit jeweils einer der zuvor beschriebenen Slipeinlagen A bis D behandelt.

Die Probandinnen tragen im Rahmen der Behandlung über einen Zeitraum von einem Monat tagsüber und nachts die jeweils zu testende Slipeinlage. Nach spätestens 8 Stunden wird die Slipeinlage durch eine neue Slipeinlage ersetzt.

Um den Behandlungserfolg mit den Slipeinlagen A bis D zu überprüfen, wird jeweils zu Beginn der Therapiephase sowie nach vier Wochen der Vaginalstatus ermittelt bzw. die Vaginalflora bestimmt. Die darin enthaltenen Bakterien- bzw. Mikroorganismen-Spezies werden auf dem Fachmann bekannte Art und Weise bestimmt. Dabei wird die Vaginalflora insbesondere im Hinblick auf die Bakterien *Gardnerella vaginalis, Atopobium vaginae, Eschericia coli,* verschiedene Arten von Streptokokken, anaerobe Bakterien, *Trichomonas vaginalis* sowie die Anzahl der Lactobacillen hin untersucht. Weiterhin wird auch die Präsenz von Hefepilzen, insbesondere der Gattung *Candida,* erfasst.

Vor Beginn der Therapie wird bei allen Probandinnen festgestellt, dass ihre Vaginalflora Streptokokken, Anaerobier und darüber hinaus teilweise *Gardnerella vaginalis,* Hefen sowie *Trichomonas vaginalis* bzw. *Atopobium vaginae* in - im Vergleich zu einer gesunden Vaginalflora - großer Zahl aufweist. Der Anteil an Lactobacillen hingegen ist bei allen Probandinnen verhältnismäßig gering bzw. deutlich verringert im Vergleich zu einer gesunden Vaginalflora.

Mit Abschluss der vierwöchigen Therapie wird der Vaginalstatus erneut erfasst:
Dabei zeigt sich bei den mit der erfindungsgemäßen Slipeinlage A behandelten Probandinnen, dass sich eine intakte Vaginalflora aufgebaut hat, welche vorwiegend Lactobacillen bzw. Milchsäuregärung betreibende Bakterien aufweist und der Anteil an Streptokokken und Anaerobiern nur äußerst gering ist.

Bei Probandinnen, welche die Vergleichsslipeinlagen B bzw. C mit Milchsäure bzw. Milchsäure produzierenden Bakterien als Einzelwirkstoffe verwendet hatten, ist die Anzahl an pathogenen Keimen, d. h. insbesondere *Trichomonas vaginalis, Atopobium vaginae, Gardnerella vaginalis* sowie Hefepilzen, leicht zurückgegangen. Die Vaginalflora kann jedoch noch nicht als intakt bezeichnet werden, d.h. der Anteil an Milchsäuregärung betreibenden Bakterien bzw. Milchsäurebakterien ist - verglichen mit normaler bzw. gesunder Vaginalflora - weiterhin zu gering.

Bei den mit der Vergleichsslipeinlage D behandelten Probandinnen hat sich der Vaginalstatus nicht signifikant geändert, d.h. die Vaginalflora konnte nicht wiederhergestellt bzw. zumindest positiv beeinflusst werden.

Aus den vorstehenden Ausführungen geht hervor, dass sich überraschenderweise mit Slipeinlagen bzw. Damenbinden, welche eine Kombination von Milchsäure produzierenden Bakterien einerseits sowie Milchsäure andererseits umfassen, eine Fehlbesiedelung der Vaginalflora insbesondere mit pathogenen Mikroorganismen, wie *Gardnerella vaginalis, Atopobium vaginae, Trichomonas vaginalis* und *Candida,* erfolgreich behandeln lässt. Die erfindungsgemäßen Wundauflagen eignen sich somit sowohl zur therapeutischen Behandlung als auch zur prophylaktischen Behandlung zur Unterstützung bzw. Wiederherstellung einer intakten Vaginalflora.

**Bezugszeichenliste:**

| | | | |
|---|---|---|---|
| 1 | Hygieneartikel | 3c | Kernschicht |
| 2 | Rückenschicht | 4 | Schutzschicht |
| 3 | Absorptionsschicht | 5 | Haftklebeschicht |
| 3a | Deckschicht | | |
| 3b | Transferschicht | | |

## Patentansprüche

1. Hygieneartikel, insbesondere zur Verwendung bei der prophylaktischen und/oder therapeutischen Behandlung von Erkrankungen des Urogenitaltrakts und/oder der Windelregion, insbesondere Vaginalinfektionen, Ureterinfektionen, Vulvitis, Windeldermatitis und/oder Ekzemen, in Form einer Damenbinde und/oder Slipeinlage,
wobei der Hygieneartikel eine Kombination ("Wirkstoffkombination") von
(a) Milchsäure produzierenden Bakterien, ausgewählt aus der Gruppe von *Lactobacillus acidophilus,* in Form von lyophilisierten und tyndallisierten Zellen ("Komponente (a)") einerseits und
(b) Milchsäure und/oder deren Salzen und/oder Estern ("Komponente (b)") in einer Menge im Bereich von 0,1 bis 1.000 mg, bezogen auf den Hygieneartikel, andererseits
aufweist.

2. Hygieneartikel nach Anspruch 1,
wobei der Hygieneartikel (1) die Milchsäure produzierenden Bakterien in einer Menge im Bereich von 10¹ bis 10³⁰ KBE (koloniebildende Einheiten), insbesondere im Bereich von 10² bis 10²⁰ KBE, vorzugsweise im Bereich von 10⁵ bis 10¹⁸ KBE, bevorzugt im Bereich von 10¹⁰ bis 10¹⁵ KBE, bezogen auf den Hygieneartikel (1), aufweist und/oder wobei der Hygieneartikel (1) Milchsäure und/oder deren Salze und/oder Ester in einer Menge im Bereich von 1 bis 500 mg, bezogen auf den Hygieneartikel (1), aufweist.

3. Hygieneartikel nach Anspruch 1 oder 2, wobei der Hygieneartikel (1) eine im Trage- bzw. Anwendungszustand dem Körper zugewandte erste Seite und eine im Trage- bzw. Anwendungs-zustand dem Körper abgewandte, insbesondere der ersten Seite gegenüberliegende zweite Seite aufweist,
wobei der Hygieneartikel die Kombination von Milchsäure produzierenden Bakterien und Milchsäure und/oder deren Salzen und/oder Estern auf der im Trage- bzw. Anwendungszustand dem Körper zugewandten ersten Seite aufweist und/oder wobei der Hygieneartikel (1) imstande ist, die Kombination von Milchsäure produzierenden Bakterien und Milchsäure und/oder deren Salzen und/oder Estern im Trage- bzw. Anwendungszustand an die im Trage- bzw. Anwendungszustand mit dem Hygieneartikel (1) in Kontakt befindlichen Körperbereich, insbesondere an den Urogenitaltrakt und/oder die Windelregion, des Trägers oder Anwenders abzugeben.

4. Hygieneartikel nach einem der vorangehenden Ansprüche, wobei der Hygieneartikel (1) schichtartig aufgebaut ist und/oder wobei der Hygieneartikel (1) mehrere Schichten aufweist.

5. Hygieneartikel nach einem der vorangehenden Ansprüche, wobei der Hygieneartikel (1) eine vorzugsweise flüssigkeitsundurchlässige Rückenschicht (2) aufweist;
insbesondere wobei die Rückenschicht (2) die im Trage- bzw. Anwendungszustand dem Körper abgewandte Seite des Hygieneartikels (1) darstellt und/oder insbesondere wobei das Material der Rückenschicht (2) ausgewählt ist aus Vliesen und/ oder Folien, insbesondere auf Basis von Polyethylenen, Polypropylenen, Polyvinylidenchloriden, Polyestern, Polystyrolen, Polyurethanen, Polyamiden, natürlichen und/oder synthetischen Kautschuken sowie deren Kombinationen, und/oder
insbesondere wobei die Rückenschicht (2) mit einer Haftklebeschicht (5) ausgerüstet ist, insbesondere wobei die Haftklebeschicht (5) ein Haft- und/oder Klebemittel enthält und/oder hieraus besteht, insbesondere wobei das Haftklebemittel ausgewählt ist aus der Gruppe von Polyurethanen, Polyvinylethern, Polyisobutylenen, Silikonen, (Meth-)-Acrylaten, Polyethylenen, Polypropylenen, Polyvinylchloriden, Polyestern, Polystyrolen sowie deren Mischungen.

6. Hygieneartikel nach einem der vorangehenden Ansprüche, wobei der Hygieneartikel (1) eine abziehbare und/oder folienbasierte Schutzschicht (4) aufweist;
insbesondere wobei die Schutzschicht (4) auf der Rückenschicht (2) des Hygieneartikels (1), insbesondere auf der im Trage- bzw. Anwendungszustand dem Körper abgewandten Seite der Rückenschicht (2), vorzugsweise auf der mit einer Haftklebeschicht (5) ausgerüsteten Seite der Rückenschicht (2), aufgebracht oder angeordnet ist, und/oder
insbesondere wobei das Material der Schutzschicht (4) ausgewählt ist aus der Gruppe von Polyestern, Polyethylenen, Polypropylenen, Polyvinylchloriden, Aluminium, Papier, insbesondere mit einer Silikon-, Polyethylen-, Fluorsilikon- und/oder Polytetrafluorethylen-Beschichtung versehenem Papier, sowie deren Mischungen.

7. Hygieneartikel nach einem der vorangehenden Ansprüche, wobei der Hygieneartikel (1) eine vorzugsweise flüssigkeitsdurchlässige, insbesondere saugfähige- und/oder absorptionsfähige Absorptionsschicht (3) aufweist;
insbesondere wobei die Absorptionsschicht (3) die im Trage- bzw. Anwendungszustand dem Körper zugewandte Seite des Hygieneartikels (1) darstellt; und/oder
insbesondere wobei die Absorptionsschicht (3) mehrlagig (mehrschichtig), insbesondere mindestens zweilagig, vorzugsweise mindestens dreilagig, ausgebildet ist, insbesondere wobei die mehrlagig ausgebildete Absorptionsschicht (3) (i) eine vorzugsweise flüssigkeitsdurchlässige und/oder saugfähige, insbesondere an die der Schutzschicht (4) abgewandten Seite der Rückenschicht (2) des Hygieneartikels angrenzende Kernschicht (3c), (ii) eine vorzugsweise flüssigkeitsdurchlässige, insbesondere an die von der Rückenschicht (2) abgewandten Seite der Kernschicht (3c) angrenzende Transferschicht (3b) und (iii) eine vorzugsweise flüssigkeitsdurchlässige, insbesondere an die von der Kernschicht (3c) abgewandten Seite der Transferschicht (3b) angrenzende Deckschicht (3a) aufweist.

8. Hygieneartikel nach einem der vorangehenden Ansprüche, wobei die Kombination der Komponenten (a) und (b) auf den Hygieneartikel (1) aufgebracht und/oder in den Hygieneartikel (1) eingebracht sind;
insbesondere wobei die Kombination der Komponenten (a) und (b) auf die Absorptionsschicht (3) aufgebracht und/oder in die Absorptionsschicht (3) eingebracht, bevorzugt in die Absorptionsschicht (3) eingebracht, ist, vorzugsweise mittels Tränken und/oder Sprühen, und/oder
insbesondere wobei die Kombination der Komponenten (a) und (b) kontinuierlich oder diskontinuierlich, vorzugsweise diskontinuierlich, bevorzugt netzförmig, auf die Absorptionsschicht (3) aufgebracht und/oder in die Absorptionsschicht (3) eingebracht, bevorzugt in die Absorptionsschicht (3) eingebracht, ist, und/oder
insbesondere wobei die Kombination der Komponenten (a) und (b) auf die Deckschicht (3a) aufgebracht und/oder in die Deckschicht (3a) eingebracht ist und/oder zwischen Deckschicht (3a) und Transferschicht (3b) eingebracht ist und/oder zwischen Transferschicht (3b) und Kernschicht (3c) eingebracht ist und/oder in Deckschicht (3a) und Transferschicht (3b) eingebracht ist.

9. Hygieneartikel nach einem der vorangehenden Ansprüche, wobei die Kombination der Komponenten (a) und (b) in einer Zusammensetzung vorliegt, insbesondere wobei die Zusammensetzung außerdem mindestens einen physiologisch kompatiblen Träger (Exzipienten) aufweist;
insbesondere wobei die Zusammensetzung Milchsäure und/oder deren Salze und/oder Ester in einer Menge im Bereich von 0,01 bis 15 Gew.-%, insbesondere im Bereich von 0,1 bis 10 Gew.-%, vorzugsweise im Bereich von 1 bis 5 Gew.-%, bevorzugt im Bereich von 2 bis 3 Gew.-%, bezogen auf die Zusammensetzung, enthält und/oder
insbesondere wobei die Zusammensetzung die Milchsäure produzierenden Bakterien in lyophilisierter und tyndallisierter Form, in einer Menge im Bereich von 0,001 bis 500 mg/g Zusammensetzung, insbesondere im Bereich von 0,005 bis 100 mg/g Zusammensetzung, vorzugsweise im Bereich von 0,01 bis 50 mg/g Zusammensetzung, bevorzugt im Bereich von 0,1 bis 10 mg/g Zusammensetzung, besonders bevorzugt im Bereich von 0,5 bis 5 mg/g Zusammensetzung, ganz besonders bevorzugt im Bereich von 0,8 bis 1,5 mg/g Zusammensetzung, aufweist und/oder
insbesondere wobei die Zusammensetzung pro g Zusammensetzung 10¹ bis 10³⁰ Zellen der Milchsäure produzierenden Bakterien, insbesondere 10² bis 10²⁰ Zellen der Milchsäure produzierenden Bakterien, vorzugsweise 10³ bis 10¹⁵ Zellen der Milchsäure produzierenden Bakterien, bevorzugt 10⁵ bis 10¹⁰ Zellen der Milchsäure produzierenden Bakterien, aufweist und/oder
insbesondere wobei die Zusammensetzung gereinigtes Wasser in einer Menge im Bereich von 1 bis 99 Gew.-%, insbesondere im Bereich von 5 bis 90 Gew.-%, vorzugsweise im Bereich von 10 bis 80 Gew.-%, bevorzugt im Bereich von 15 bis 70 Gew.-%, besonders bevorzugt im Bereich von 20 bis 65 Gew.-%, ganz besonders bevorzugt im Bereich von 30 bis 60 Gew.-%, bezogen auf die Zusammensetzung, aufweist und/oder wobei die Zusammensetzung gereinigtes Wasser in einer Menge von höchstens 90 Gew.-%, vorzugsweise höchstens 80 Gew.-%, bevorzugt höchstens 70 Gew.-%, besonders bevorzugt höchstens 60 Gew.-%, bezogen auf die Zusammensetzung, aufweist.

10. Hygieneartikel nach Anspruch 9,
wobei der Träger ausgewählt ist aus der Gruppe von Wasser, Ethanol, Isopropanol, Ethylcarbonat, Ethylacetat, Benzylalkohol, Benzylbenzoat, Propylenglycol, 1,3-Butylglycol, Wollfett, Baumwollsaatöl, Vaseline, Glycerinfettsäureestern, Polyethylenglykolen, Paraffinen sowie deren Mischungen und Kombinationen und/oder wobei die Zusammensetzung den Träger in einer Menge im Bereich von 0,1 bis 50 Gew.-%, insbesondere im Bereich von 1 bis 40 Gew.-%, vorzugsweise im Bereich von 5 bis 30 Gew.-%, bevorzugt im Bereich von 10 bis 20 Gew.-%, bezogen auf die Zusammensetzung, enthält.

11. Hygieneartikels nach einem der vorangehenden Ansprüche zur Verwendung bei der prophylaktischen und/oder therapeutischen Behandlung von Erkrankungen des Urogenitaltrakts und/oder der Windelregion, insbesondere von Vaginalinfektionen, Ureterinfektionen, Vulvitis, Windeldermatitis und/oder Ekzemen.

12. Verwendung einer Kombination ("Wirkstoffkombination") von
(a) Milchsäure produzierende Bakterien, ausgewählt aus der Gruppe von *Lactobacillus acidophilus,* in Form von lyophilisierten und tyndallisierten Zellen ("Komponente (a)") einerseits und
(b) Milchsäure und/oder deren Salzen und/oder Estern ("Komponente (b)") in einer Menge im Bereich von 0,1 bis 1.000 mg, bezogen auf den Hygieneartikel, andererseits
zur Ausrüstung eines Hygieneartikels, insbesondere nach einem der Ansprüche 1 bis 10, in Form einer Damenbinde und/oder Slipeinlage, mit keimhemmenden Eigenschaften, insbesondere eines Hygieneartikels zur Verwendung bei der prophylaktischen und/oder therapeutischen Behandlung von Erkrankungen des Urogenitaltrakts und/oder der Windelregion, insbesondere Vaginalinfektionen, Ureterinfektionen, Vulvitis, Windeldermatitis und/oder Ekzemen.

13. Kombination ("Wirkstoffkombination") zur Ausrüstung eines Hygieneartikels, insbesondere nach einem der Ansprüche 1 bis 10,
mit keimhemmenden Eigenschaften,
wobei die Kombination, insbesondere in jeweils (therapeutisch) wirksamen Mengen und/oder insbesondere zusammen mit einem kompatiblen Träger,
(a) Milchsäure produzierende Bakterien, ausgewählt aus der Gruppe von *Lactobacillus acidophilus,* in Form von lyophilisierten und tyndallisierten Zellen ("Komponente (a)") einerseits und
(b) Milchsäure und/oder deren Salze und/oder Ester in einer Menge im Bereich von 0,1 bis 1.000 mg, bezogen auf den Hygieneartikel, ("Komponente (b)") andererseits enthält.

14. Kombination ("Wirkstoffkombination") zur Verwendung bei der prophylaktischen und/oder therapeutischen Behandlung von Erkrankungen des Urogenitaltrakts und/oder der Windelregion, insbesondere Vaginalinfektionen, Ureterinfektionen, Vulvitis, Windeldermatitis und/oder Ekzemen,
wobei die Kombination, insbesondere in jeweils (therapeutisch) wirksamen Mengen und/oder insbesondere zusammen mit einem kompatiblen Träger,
(a) Milchsäure produzierende Bakterien, ausgewählt aus der Gruppe von *Lactobacillus acidophilus,* in Form von lyophilisierten und tyndallisierten Zellen ("Komponente (a)") einerseits und
(b) Milchsäure und/oder deren Salze und/oder Ester in einer Menge im Bereich von 0,1 bis 1.000 mg, bezogen auf den Hygieneartikel, ("Komponente (b)") andererseits enthält.

## Claims

1. A hygiene article, in particular for use in the prophylactic and/or therapeutic treatment of diseases of the urogenital tract and/or the nappy area, particularly vaginal infections, ureteral infections, vulvitis, nappy rash and/or eczemas, in the form of a sanitary napkin and/or panty liner,
wherein the hygiene article comprises a combination ("active ingredient combination") of
(a) lactic-acid producing bacteria selected from the group of *Lactobacillus acidophilus* in the form of freeze-dried and tyndallized cells ("components (a)") on the one hand and
(b) lactic acid and/or its salts and/or esters ("component (b)") in a quantity in the range of 0.1 to 1000 mg based on the hygiene article on the other.

2. The hygiene article according to claim 1,
wherein the hygiene article (1) comprises the lactic acid producing bacteria in a quantity in a range of 10¹ to 10³⁰ CFU (colony-forming units), in particular in a range of 10² to 10²⁰ CFU, preferably in a range of 10⁵ to 10¹⁸ CFU, more preferably in a range of 10¹⁰ to 10¹⁵ CFU, based on the hygiene article (1) and/or
wherein the hygiene article (1) comprises lactic acid and/or its salts and/or esters in a quantity in the range of 1 to 500 mg, based on the hygiene article (1).

3. The hygiene article according to claim 1 or 2, wherein the hygiene article (1) has a first side turned toward the body in the carrying or application state and a second side, in particular opposite the first side, facing away from the body in the carrying or application state,
wherein the hygiene article has the combination of lactic acid producing bacteria and lactic acid and/or its salts and/or esters on the first side facing the body in the carrying or application state and/or wherein the hygiene article (1) is able to dispense the combination of lactic acid producing bacteria and lactic acid and/or its salts and/or esters, in the carrying or application state, to the body region in contact with the hygiene article (1) in the carrying or application state, in particular to the urogenital tract and or the nappy area, of the carrier or applicator.

4. The hygiene article according to one of the previous claims, wherein the hygiene article (1) is structured in layers and/or wherein the hygiene article (1) comprises several layers.

5. The hygiene article according to one of the previous claims, wherein the hygiene article (1) comprises a preferably liquid-permeable backing layer (2);
in particular wherein the backing layer (2) constitutes the side of the hygiene article (1) facing away from the body in the carrying or application state and/or
in particular wherein the material of the backing layer (2) is selected from nonwovens and/or films, in particular based on polyethylenes, polypropylenes, polyvinylidene chlorides, polyesters, polystyrenes, polyurethanes, polyamides, natural and/or synthetic rubbers, and combinations thereof, and/or
in particular wherein the backing layer (2) is equipped with an adhesive layer (5), in particular wherein the adhesive layer (5) contains an adhesive and/or bonding agent and/or consists thereof, in particular wherein the adhesive is selected from the group consisting of polyurethanes, polyvinyl ethers, polyisobutylenes, silicones, (meth)acrylates, polyethylenes, polypropylenes, polyvinyl chlorides, polyesters, polystyrenes, and mixtures thereof.

6. The hygiene article according to one of the preceding claims, wherein the hygiene article (1) comprises a peelable and/or film-based protective layer (4);
in particular wherein the protective layer (4) is applied or arranged on the backing layer (2) of the hygiene article (1), in particular on the side of the backing layer (2) facing away from the body in the carrying or application state, preferably on the side of the backing layer (2) equipped with an adhesive layer (5), and/or
in particular wherein the material of the protective layer (4) is selected from the group consisting of polyesters, polyethylenes, polypropylenes, polyvinyl chlorides, aluminium, paper, especially paper provided with a silicone, polyethylene, fluorosilicone and/or polytetrafluoroethylene coating, and mixtures thereof.

7. The hygiene article according to one of the preceding claims, wherein the hygiene article (1) comprises a preferably liquid-permeable, in particular suction-capable and/or absorption-capable absorption layer (3);
in particular wherein the absorption layer (3) constitutes the side of the hygiene article (1) facing toward the body in the carrying or application state; and/or
in particular wherein the absorption layer (3) is configured in several layers (multi-layered), in particular at least two-layered, preferably at least three-layered, in particular wherein the absorption layer (3) in several layers comprises (i) a preferably liquid-permeable and/or suction-capable core layer (3c) abutting the side of the backing layer (2) of the hygiene article facing away from the protective layer (4); (ii) a preferably liquid-permeable transfer layer (3b) abutting the side of the core layer (3c) facing away from the backing layer (2); and (iii) a preferably liquid-permeable cover layer (3a) abutting the side of the transfer layer (3b) facing away from the core layer (3c).

8. The hygiene article according to one of the preceding claims, wherein the combination of the components (a) and (b) is applied on the hygiene article (1) and/or inserted in the hygiene article (1);
in particular wherein the combination of components (a) and (b) is applied on the absorption layer (3) and/or inserted in the absorption layer (3), preferably inserted in the absorption layer (3), preferably by soaking and/or spraying, and/or
in particular wherein the combination of the components (a) and (b) is applied continuously or discontinuously, preferably discontinuously, preferably in a reticulated manner, on the absorption layer (3) and/or inserted in the absorption layer (3), preferably inserted in the absorption layer (3), and/or
in particular wherein the combination of the components (a) and (b) are applied on the cover layer (3a) and/or is inserted in the cover layer (3a) and/or is inserted between the cover layer (3a) and transfer layer (3b) and/or is inserted between the transfer layer (3b) and core layer (3c), and/or is inserted in the cover layer (3a) and transfer layer (3b).

9. The hygiene article according to one of the preceding claims, wherein the combination of components (a) and (b) is present in a composition, in particular wherein the composition in addition comprises at least one physiologically compatible carrier (excipient);
in particular wherein the composition contains lactic acid and/or its salts and/or esters in a quantity in the range of 0.01 to 15 wt%, in particular in the range of 0.1 to 10 wt%, preferably in the range of 1 to 5 wt%, more preferably in the range of 2 to 3 wt% with respect to the composition and/or
in particular wherein the composition comprises the lactic acid producing bacteria in freeze-dried and tyndallized form, in a quantity in the range of a 0.001 to 500 mg/g of composition, in particular in the range of 0.005 to 100 mg/g of composition; preferably in the range of 0.01 to 50 mg/g of composition; more preferably in the range of 0.1 to 10 mg/g of composition; especially preferably in the range of 0.5 to 5 mg/g of composition, most preferably in the range of 0.8 to 1.5 mg/g of composition, and or
in particular wherein the composition per g of composition comprises 10¹ to 10³⁰ cells of lactic acid producing bacteria, in particular 10² to 10²⁰ cells of lactic acid producing bacteria, preferably 10³ to 10¹⁵ cells of lactic acid producing bacteria, more preferably 10⁵ to 10¹⁰ cells of lactic acid producing bacteria, and/or
in particular wherein the composition comprises purified water in a quantity in the range of 1 to 99 wt%, in particular in the range of 5 to 90 wt%, preferably in the range of 10 to 80 wt%, more preferably in the range of 15 to 70 wt%, especially preferably in the range of 20 to 65 wt%, most preferably in the range of 30 to 60 wt% with respect to the composition, and/or wherein the composition comprises purified water in a quantity of at most 90 wt%, preferably at most 80 wt%, more preferably at most 70 wt%, and especially preferably at most 60 wt% with respect to the composition.

10. The hygiene article according to claim 9,
wherein the carrier is selected from the group consisting of water, ethanol, isopropanol, ethyl carbonate, ethyl acetate, benzyl alcohol, benzyl benzoate, propylene glycol, 1,3-butyl glycol, lanolin, cottonseed oil, vaseline, glycerol fatty acid esters, polyethylene glycols, paraffins, and mixtures and combinations thereof, and/or
wherein the composition contains the carrier in a quantity in the range of 0.1 to 50 wt%, in particular in the range of 1 to 40 wt%, preferably in the range of 5 to 30 wt%, more preferably in the range of 10 to 20 wt%, with respect to the composition.

11. The hygiene article according to one of the previous claims for use in the prophylactic and/or therapeutic treatment of diseases of the urogenital tract and/or the nappy area, in particular of vaginal infections, ureteral infections, vulvitis, nappy rash and/or eczemas.

12. Use of a combination ("active ingredient combination") of
(a) lactic acid producing bacteria selected from the group of *Lactobacillus acidophilus* in the form of freeze-dried and tyndallized cells ("component (a)") on the one hand and
(b) lactic acid and/or its salts and/or esters ("component (b)") in a quantity in the range of 0.1 to 1000 mg based on the hygiene article
for equipping a hygiene article, in particular according to any one of claims 1 to 10, in the form of a sanitary napkin and/or panty liner, with bacteria-inhibiting properties, in particular a hygiene article for use in the prophylactic and/or therapeutic treatment of diseases of the urogenital tract and/or of the nappy region, in particular vaginal infections, ureteral infections, vulvitis, nappy rash and/or eczemas.

13. A combination ("active ingredient combination") for equipping a hygiene article, in particular according to any one of claims 1 to 10, with bacteria-inhibiting properties, wherein the combination, in particular in (therapeutically) effective quantities and/or in particular together with a compatible carrier,
(a) contains lactic acid producing bacteria selected from the group of *Lactobacillus acidophilus* in the form of freeze-dried and tyndallized cells ("component (a)") on the one hand and
(b) lactic acid and/or its salts and/or esters in a quantity in the range of 0.1 to 1000 mg, based on the hygiene article, ("component (b)") on the other hand.

14. A combination ("active ingredient combination") for use in the prophylactic and/or therapeutic treatment of diseases of the urogenital tract and/or of the nappy region, in particular vaginal infections, ureteral infections, vulvitis, nappy rash and/or eczemas, wherein the combination, in particular in (therapeutically) effective quantities and/or in particular together with a compatible carrier,
(a) contains lactic acid producing bacteria selected from the group of *Lactobacillus acidophilus* in the form of freeze-dried and tyndallized cells ("component (a)") on the one hand and
(b) lactic acid and/or its salts and/or esters in a quantity in the range of 0.1 to 1000 mg, based on the hygiene article, ("component (b)") on the other hand.

## Revendications

1. Article d'hygiène, destiné en particulier à être utilisé pour le traitement prophylactique et/ou thérapeutique de maladies du tractus urogénital et/ou du siège, en particulier des infections vaginales, des infections de l'uretère, de la vulvite, de la dermite du siège et/ou de l'eczéma, sous la forme d'une protection hygiénique et/ou d'un protège-slip,
l'article d'hygiène présentant une combinaison (« combinaison de substances actives »)
(a) de bactéries produisant de l'acide lactique choisies dans le groupe de *Lactobacillus acidophilus,* sous la forme de cellules lyophilisées et tyndallisées (« composant (a) ») d'une part et
(b) d'acide lactique et/ou ses sels et/ou esters (« composant (b) ») dans une quantité située dans la plage allant de 0,1 à 1000 mg, rapportée à l'article d'hygiène
d'autre part.

2. Article d'hygiène selon la revendication 1,
l'article d'hygiène (1) présentant les bactéries produisant de l'acide lactique dans une quantité située dans la plage allant de 10¹ à 10³⁰ UFC (unités formant colonies), en particulier dans la plage allant de 10² à 10²⁰ UFC, de préférence dans la plage allant de 10⁵ à 10¹⁸ UFC, de manière préférée dans la plage allant de 10¹⁰ à 10¹⁵ UFC, rapportée à l'article d'hygiène (1), et/ou
l'article d'hygiène (1) présentant de l'acide lactique et/ou ses sels et/ou esters dans une quantité située dans la plage allant de 1 à 500 mg, rapportée à l'article d'hygiène (1).

3. Article d'hygiène selon la revendication 1 ou 2, l'article d'hygiène (1) présentant une première face tournée vers le corps à l'état porté ou utilisé, et une seconde face éloignée du corps, en particulier opposée à la première face, à l'état porté ou utilisé,
l'article d'hygiène présentant la combinaison de bactéries produisant de l'acide lactique et d'acide lactique et/ou ses sels et/ou esters sur la première face tournée vers le corps à l'état porté ou utilisé et/ou l'article d'hygiène (1) étant en mesure de céder à l'état porté ou utilisé la combinaison de bactéries produisant de l'acide lactique et d'acide lactique et/ou ses sels et/ou esters à la zone corporelle se trouvant à l'état porté ou utilisé en contact avec l'article d'hygiène (1), en particulier au tractus urogénital et/ou au siège.

4. Article d'hygiène selon l'une quelconque des revendications précédentes, l'article d'hygiène (1) étant conçu sous forme de couches et/ou l'article d'hygiène (1) présentant plusieurs couches.

5. Article d'hygiène selon l'une quelconque des revendications précédentes, l'article d'hygiène (1) présentant une couche support (2), de préférence imperméable aux liquides;
en particulier, la couche support (2) représentant la face de l'article d'hygiène (1) éloignée du corps à l'état porté ou utilisé et/ou
en particulier, le matériau de la couche support (2) étant choisi parmi des non-tissés et/ou des feuilles, en particulier à base de polyéthylènes, de polypropylènes, de polychlorures de vinylidène, de polyesters, de polystyrènes, de polyuréthannes, de polyamides, de caoutchoucs naturels et/ou synthétiques et de leurs combinaisons, et/ou en particulier, la couche support (2) étant munie d'une couche adhésive de contact (5), en particulier la couche adhésive de contact (5) contenant un agent adhésif et/ou collant et/ou étant constituée de ceux-ci, en particulier, l'agent adhésif de contact étant choisi dans le groupe des polyuréthannes, des éthers polyvinyliques, des polyisobutylènes, des silicones, des (méth)acrylates, des polyéthylènes, des polypropylènes, des polychlorures de vinyle, des polyesters, des polystyrènes et de leurs mélanges.

6. Article d'hygiène selon l'une quelconque des revendications précédentes, l'article d'hygiène (1) présentant une couche protectrice (4) détachable et/ou à base d'une feuille;
en particulier, la couche protectrice (4) étant appliquée ou agencée sur la couche support (2) de l'article d'hygiène (1), en particulier sur la face de la couche support (2) éloignée du corps à l'état porté ou utilisé, de préférence sur la face de la couche support (2) munie d'une couche adhésive de contact (5), et/ou
en particulier, le matériau de la couche protectrice (4) étant choisi dans le groupe des polyesters, des polyéthylènes, des polypropylènes, des polychlorures de vinyle, de l'aluminium, du papier, en particulier d'un papier muni d'un couchage de silicone, de polyéthylène, de silicone fluorée et/ou de polytétrafluoréthylène, et de leurs mélanges.

7. Article d'hygiène selon l'une quelconque des revendications précédentes, l'article d'hygiène (1) présentant une couche absorbante (3) de préférence perméable aux liquides, en particulier capable d'effectuer une absorption ;
en particulier, la couche absorbante (3) représentant la face de l'article d'hygiène (1) tournée vers le corps à l'état porté ou utilisé ; et/ou
en particulier, la couche absorbante (3) étant multicouche, en particulier constituée d'au moins deux couches, de préférence d'au moins trois couches, en particulier la couche absorbante (3) conçue de manière multicouche présentant (i) une couche de coeur (3c) de préférence perméable aux fluides et/ou absorbante, en particulier adjacente à la face de la couche support (2) de l'article d'hygiène qui est éloignée de la couche protectrice (4), (ii) une couche de transfert (3b) de préférence perméable aux liquides, en particulier adjacente à la face de la couche de coeur (3c) éloignée de la couche support (2) et (iii) une couche supérieure (3a) de préférence perméable aux liquides, en particulier adjacente à la face de la couche de transfert (3b) éloignée de la couche de coeur (3c).

8. Article d'hygiène selon l'une quelconque des revendications précédentes, la combinaison des composants (a) et (b) étant appliquée sur l'article d'hygiène (1) et/ou incorporée à l'article d'hygiène (1) ;
en particulier, la combinaison des composants (a) et (b) étant appliquée sur la couche absorbante (3) et/ou incorporée à la couche absorbante (3), de préférence incorporée à la couche absorbante (3), de préférence par imprégnation et/ou pulvérisation, et/ou en particulier, la combinaison des composants (a) et (b) étant appliquée sur la couche absorbante (3) de manière continue ou discontinue, de préférence discontinue, de préférence sous forme de réseau et/ou incorporée à la couche absorbante (3), de préférence incorporée à la couche absorbante (3), et/ou
en particulier, la combinaison des composants (a) et (b) étant appliquée sur la couche supérieure (3a) et/ou incorporée à la couche supérieure (3a) et/ou incorporée entre la couche supérieure (3a) et la couche de transfert (3b) et/ou incorporée entre la couche de transfert (3b) et la couche de coeur (3c) et/ou incorporée dans la couche supérieure (3a) et la couche de transfert (3b).

9. Article d'hygiène selon l'une quelconque des revendications précédentes, la combinaison des composants (a) et (b) étant présente dans une composition, en particulier la composition présentant en outre au moins un véhicule (excipient) physiologiquement compatible ;
en particulier, la composition contenant de l'acide lactique et/ou ses sels et/ou esters dans une quantité située dans la plage allant de 0,01 à 15 % en poids, en particulier dans la plage allant de 0,1 à 10 % en poids, de préférence dans la plage allant de 1 à 5 % en poids, de manière préférée dans la plage allant de 2 à 3 % en poids, rapportée à la composition, et/ou
en particulier, la composition présentant les bactéries produisant de l'acide lactique sous la forme lyophilisée et tyndallisée, dans une quantité située dans la plage allant de 0,001 à 500 mg/g de composition, en particulier dans la plage allant de 0,005 à 100 mg/g de composition, de préférence dans la plage allant de 0,01 à 50 mg/g de composition, de manière préférée dans la plage allant de 0,1 à 10 mg/g de composition, de manière particulièrement préférée dans la plage allant de 0,5 à 5 mg/g de composition, de manière tout particulièrement préférée dans la plage allant de 0,8 à 1,5 mg/g de composition, et/ou en particulier, la composition présentant, par gramme de composition, de 10¹ à 10³⁰ cellules de bactéries produisant de l'acide lactique, en particulier de 10² à 10²⁰ cellules de bactéries produisant l'acide lactique, de préférence de 10³ à 10¹⁵ cellules de bactéries produisant de l'acide lactique, de préférence de 10⁵ à 10¹⁰ cellules de bactéries produisant de l'acide lactique, et/ou
en particulier, la composition présentant de l'eau purifiée dans une quantité située dans la plage allant de 1 à 99 % en poids, en particulier dans la plage allant de 5 à 90 % en poids, de préférence dans la plage allant de 10 à 80 % en poids, de manière préférée dans la plage allant de 15 à 70 % en poids, de manière particulièrement préférée dans la plage allant de 20 à 65 % en poids, de manière tout particulièrement préférée dans la plage allant de 30 à 60 % en poids, rapportée à la composition, et/ou la composition présentant de l'eau purifiée dans une quantité d'au plus 90 % en poids, de préférence d'au plus 80 % en poids, de manière préférée d'au plus 70 % en poids, de manière particulièrement préférée d'au plus 60 % en poids, rapportée à la composition.

10. Article d'hygiène selon la revendication 9,
le véhicule étant choisi dans le groupe de l'eau, de l'éthanol, de l'isopropanol, du carbonate d'éthyle, de l'acétate d'éthyle, de l'alcool benzylique, du benzoate de benzyle, du propylène glycol, du 1,3-butylglycol, de la lanoline, de l'huile de coton, de la vaseline, des esters d'acides gras du glycérol, des polyéthylène glycols, des paraffines et de leurs mélanges et combinaisons et/ou
la composition contenant le véhicule dans une quantité située dans la plage allant de 0,1 à 50 % en poids, en particulier dans la plage allant de 1 à 40 % en poids, de préférence dans la plage allant de 5 à 30 % en poids, de manière préférée dans la plage allant de 10 à 20 % en poids, rapportée à la composition.

11. Article d'hygiène selon l'une quelconque des revendications précédentes, destiné à être utilisé pour le traitement prophylactique et/ou thérapeutique de maladies du tractus urogénital et/ou du siège, en particulier des infections vaginales, des infections de l'uretère, de la vulvite, de la dermite du siège et/ou de l'eczéma.

12. Utilisation d'une combinaison (« combinaison de substances actives »)
(a) des bactéries produisant de l'acide lactique choisies dans le groupe de *Lactobacillus acidophilus,* sous la forme de cellules lyophilisées et tyndallisées (« composant (a) ») d'une part et
(b) d'acide lactique et/ou ses sels et/ou esters (« composant (b) ») dans une quantité située dans la plage allant de 0,1 à 1000 mg, rapportée à l'article d'hygiène, d'autre part
pour équiper un article d'hygiène, en particulier selon l'une quelconque des revendications 1 à 10, sous la forme d'une protection hygiénique et/ou d'un protège-slip, doté de propriétés bactériostatiques, en particulier un article d'hygiène destiné à être utilisé pour le traitement prophylactique et/ou thérapeutique de maladies du tractus urogénital et/ou du siège, en particulier des infections vaginales, des infections de l'uretère, de la vulvite, de la dermite du siège et/ou de l'eczéma.

13. Combinaison (« combinaison de substances actives ») destinée à équiper un article d'hygiène, en particulier selon l'une quelconque des revendications 1 à 10, doté de propriétés bactériostatiques,
la combinaison contenant, en particulier dans des quantités respectivement (thérapeutiquement) actives et/ou en particulier conjointement avec un véhicule compatible,
(a) des bactéries produisant de l'acide lactique choisies dans le groupe de *Lactobacillus acidophilus,* sous la forme de cellules lyophilisées et tyndallisées (« composant (a) ») d'une part et
(b) de l'acide lactique et/ou ses sels et/ou esters (« composant (b) ») dans une quantité située dans la plage allant de 0,1 à 1000 mg, rapportée à l'article d'hygiène d'autre part.

14. Combinaison (« combinaison de substances actives ») destinée à être utilisée pour le traitement prophylactique et/ou thérapeutique de maladies du tractus urogénital et/ou du siège, en particulier des infections vaginales, des infections de l'uretère, de la vulvite, de la dermite du siège et/ou de l'eczéma,
la combinaison contenant, en particulier dans des quantités respectivement (thérapeutiquement) actives et/ou en particulier conjointement avec un véhicule compatible,
(a) des bactéries produisant de l'acide lactique choisies dans le groupe de *Lactobacillus acidophilus,* sous la forme de cellules lyophilisées et tyndallisées (« composant (a) ») d'une part et
(b) de l'acide lactique et/ou ses sels et/ou esters (« composant (b) ») dans une quantité située dans la plage allant de 0,1 à 1000 mg, rapportée à l'article d'hygiène, d'autre part.
